(19) Europäisches Patentamt European Patent Office Office européen des brevets

(11) **EP 4 563 591 A1**

(12) **EUROPEAN PATENT APPLICATION**
published in accordance with Art. 153(4) EPC

(43) Date of publication:
04.06.2025 Bulletin 2025/23

(21) Application number: 23847053.8

(22) Date of filing: 31.07.2023

(51) International Patent Classification (IPC):
*C07K 14/00* (2006.01)    *C07K 14/47* (2006.01)
*C12N 15/62* (2006.01)    *A61K 39/00* (2006.01)
*A61P 37/00* (2006.01)

(52) Cooperative Patent Classification (CPC):
A61K 39/00; A61P 37/00; C07K 14/00;
C07K 14/47; C12N 15/62

(86) International application number:
PCT/KR2023/011095

(87) International publication number:
WO 2024/025397 (01.02.2024 Gazette 2024/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.07.2022 KR 20220095094

(71) Applicant: LG Chem, Ltd.
Yeongdeungpo-gu
Seoul 07336 (KR)

(72) Inventors:
• CHO, Seong Je
  Daejeon 34122 (KR)
• MIN, Kyeongsik
  Daejeon 34122 (KR)
• KIM, Dae Hee
  Daejeon 34122 (KR)
• KIM, Youngmok
  Daejeon 34122 (KR)

• SHIN, Do Woon
  Daejeon 34122 (KR)
• RYU, Su Jeong
  Daejeon 34122 (KR)
• KIM, Han Byul
  Daejeon 34122 (KR)
• KIM, Da Eun
  Daejeon 34122 (KR)
• JANG, Sung Woong
  Daejeon 34122 (KR)
• PARK, Jun Gyu
  Daejeon 34122 (KR)

(74) Representative: Hoffmann Eitle
Patent- und Rechtsanwälte PartmbB
Arabellastraße 30
81925 München (DE)

Remarks:
The complete document including Reference
Table(s) and the Sequence Listing(s) can be
downloaded from the EPO website

(54) **RECOMBINANT FUSION PROTEIN FOR ANTIGEN DELIVERY AND USES THEREOF**

(57) The present invention relates to a fusion protein comprising a peptide antigen containing a T cell epitope, a first carrier protein linked to the N-terminus of the peptide antigen, and a second carrier protein linked to the C-terminus of the peptide antigen; a nucleic acid molecule encoding the fusion protein; an expression vector containing the nucleic acid molecule; a cell transformed with the expression vector; and an immunogenic composition comprising the fusion protein, the nucleic acid molecule, the expression vector, or the cell.

EP 4 563 591 A1

【FIG. 5】

Anti-tumor effect in CT26 Syngeneic model

## Description

[TECHNICAL FIELD]

Related are a fusion protein, comprising a peptide antigen containing a T cell epitope, and a carrier protein linked to the N-terminus or C-terminus of the peptide antigen or both of them; a nucleic acid molecule encoding the fusion protein; an expression vector comprising the nucleic acid molecule; a cell transformed with the expression vector; and an immunogenic composition comprising the fusion protein, nucleic acid molecule, expression vector, or cell.

[BACKGROUNT ART]

Technologies have been developed to deliver antigens in various forms to induce T cell immunity, but there are advantages and disadvantages depending on the form. When an antigen is delivered in a peptide form, there is a disadvantage of difficult synthesis and purification and low exposure due to solubility and short half-life depending on various antigen sequences. When an antigen is delivered in an mRNA form, there is an advantage of consistency of preparation, but there is a disadvantage such that formulation due to instability in a body is necessary and delivering a certain amount of antigen (in vivo constant expression of various sequences) is uncertain. When an antigen is delivered in a DNA form, there is a disadvantage in that in vivo stability is better than mRNA, but it should be delivered into a nucleus, and antigen expression efficiency is low and DNA may be inserted into genome. A virus vector has an advantage of delivery into a body and high immunogenicity, but has a disadvantage of reducing drug efficacy due to an anti-drug antibody in booster vaccination due to production of strong antibodies against the virus surface.

Accordingly, development of a vaccine capable of delivering an antigen into a body more effectively and inducing a strong immune response is required.

[Prior art]

[Patent document]

(Patent document 1) U.S. Patent No. 10023657 (2018.7.17.)

[DISCLOSURE]

[TECHNICAL PROBLEM]

The present invention provides an antigen delivery system which improves expression, physical properties, stability and/or immunogenicity of an antigen, and improves delivery of an antigen into an immune response cell. In addition, the present invention provides an antigen delivery system capable of preparing, expressing, purifying and delivering sequences of various antigens, and capable of inducing a stronger immune response in vivo.

One embodiment provides a fusion protein comprising a peptide antigen, and a carrier protein linked to the N-terminus, C-terminus, or both of the peptide antigen. A preferable embodiment, provides a fusion protein, comprising a peptide antigen, a first carrier protein linked to the N-terminus of the peptide antigen, and a second carrier protein linked to the C-terminus of the peptide antigen.

Another embodiment, provides a nucleic acid molecule encoding the fusion protein.

Other embodiment, provides an expression vector comprising the nucleic acid molecule.

Other embodiment, provides a cell transformed with the expression vector.

Other embodiment, provides a vaccine composition or an immunogenic composition comprising the fusion protein, nucleic acid molecule, expression vector, or cell.

Other embodiment, provides a composition for prevention or treatment of disease comprising the fusion protein, nucleic acid molecule, expression vector, or cell.

[TECHNICAL SOLUTION]

According to one aspect of the present invention provides a fusion protein comprising a peptide antigen, and a carrier protein linked to the N-terminus, C-terminus, or both of the peptide antigen.

One embodiment provides a fusion protein, comprising a peptide antigen, a first carrier protein linked to the N-terminus of the peptide antigen, and a second carrier protein linked to the C-terminus of the peptide antigen.

As one embodiment, in the fusion protein, an affinity tag may be linked to the N-terminus, C-terminus, or both thereof.

As one embodiment, a linker may be present between the peptide antigen and the first carrier protein, or a linker may be present between the peptide antigen and the second carrier protein, or both.

As one embodiment, the peptide antigen may contain a T cell epitope.

As one embodiment, the peptide antigen may comprise a T cell epitope, derived from a tumor antigen, an antigen of an infection source, an autoantigen, or an allergy-inducing antigen.

As one embodiment, the carrier protein may be a protein which improves recombinant expression of the peptide antigen or improves purification of the peptide antigen.

As one embodiment, the first carrier protein may be a protein which improves recombinant expression of the peptide antigen, and the second carrier protein may be a protein which improves improvement of the peptide antigen.

As one embodiment, the tumor antigen may include a tumor-associated antigen (TAA), a tumor-specific antigen (TSA) or a tumor-derived neoantigen.

As one embodiment, the tumor-derived neoantigen may comprise a mutation expressed specifically in a cancer cell.

As one embodiment, the tumor-associated antigen (TAA) may be CT (Cancer-testis) antigen, EGFR, M12, M20, M21, M30, M44, Ova, Melan-A, PSMA(Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E), GAGE (G antigen), BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, N-RAS, K-RAS, RCAS1, $\alpha$-fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA.

As one embodiment, the antigen of the infection source may be an antigen derived from a virus, a bacterium, a parasite, or a fungus.

As one embodiment, the first carrier protein and the second carrier protein may be same or different, and may be NDPK (nucleoside diphosphate kinase B), CSTA(Cystatin-A), Trx(Thioredoxin), RPL7Am (50S ribosomal protein L7Ae), Samp2a (Small archaeal modifier protein 2), TE (Tenascin), TM1112 *(Thermotoga maritima* Cupin_3 domain-containing protein), TrxA (Thioredoxin 1), TTrx (*Thermosipho africanus* Thioredoxin), or PSBD(peripheral subunit-binding domain), or fragments thereof, or at least two kinds thereof, respectively.

As one embodiment, the affinity tag may be His or streptavidin.

As one embodiment, the linker may be (GS)n, (G$_2$S)n, (G$_3$S)n, (G$_4$S)n, Gn, LE, SSGG or GGGGSGGGGG (wherein, G is Gly, and S is Ser, and L is Leu, and E is Glu, and n is an integer of at least 1).

As one embodiment, the fusion protein may be in a size of 30kDa or less.

According to another aspect of the present invention, a nucleic acid molecule encoding the fusion protein is provided.

According to other aspect of the present invention, an expression vector comprising the nucleic acid molecule is provided.

According to other aspect of the present invention, a cell transformed with the expression vector is provided.

According to other aspect of the present invention, a vaccine composition comprising the fusion protein, nucleic acid molecule, expression vector, or cell is provided.

According to other aspect of the present invention, an immunogenic composition comprising the fusion protein, nucleic acid molecule, expression vector, or cell is provided.

As one embodiment, the compositions may further comprise an adjuvant.

According to other aspect of the present invention, a method for causing, inducing, and/or promoting an immune response against an antigen, comprising administering an effective dose of the fusion protein, vaccine composition and/or immunogenic composition into a patient is provided.

According to other aspect of the present invention, a method for preventing, improving and/or treating cancer, infectious disease, autoimmune disease, or allergic disease, comprising administering an effective dose of the fusion protein, vaccine composition and/or immunogenic composition into a patient is provided.

According to other aspect of the present invention, a use for causing, inducing or promoting an immune response against an antigen of the fusion protein, or a use for use in preparation of a composition for causing, inducing or promoting an immune response is provided.

According to other aspect of the present invention, a use for preventing, improving and/or treating cancer, infectious disease, autoimmune disease, or allergic disease, or a use for using in preparation of a composition for preventing, improving and/or treating cancer, infectious disease, autoimmune disease, or allergic disease, of the fusion protein is provided.

Hereinafter, the present invention will be descried in more detail.

In the present description (including claims), when the terms of "comprise, comprises" , "comprised" or "comprising" is used, it is interpreted that they are specifying presence of described characteristics, integers, steps or elements, but it should be interpreted not as excluding presence of one or more other characteristics, integers, steps, elements or groups thereof.

In the present description, description of documents, laws, materials, devices, and articles, and the like, are included only for the purpose of providing context for the present invention. All or a part thereof do not form a part based on the prior art or suggest or exhibit what was common general knowledge in the field to which the present invention belongs prior to the priority date of each claim of the present application.

One example of the present application relates to a fusion protein comprising a peptide antigen, and a carrier protein linked to the N-terminus, C-terminus, or both, of the peptide antigen. As a preferable embodiment, it relates to a fusion protein, comprising a peptide antigen, a first carrier protein linked to the N-terminus of the peptide antigen, and a second carrier protein linked to the C-terminus of the peptide antigen.

The term, "antigen" refers to any molecule inducing an immune response in a subject. As one example, the antigen may refer to any molecule containing an epitope, which can be recognized by a T cell receptor and/or a B cell receptor, and stimulate an immune response, particularly, a T cell response and/or a B cell response in a subject.

The term, "epitope" refers to namely, a region of an antigen interacting with a T-cell receptor and/or a B-cell receptor.

As one preferable example, the peptide antigen of the present application may contain a T cell epitope. For example, the peptide antigen of the present application may comprise an MHC class I and/or II binding motif. As a specific example, the peptide antigen of the present application, may contain a CD4+ T cell epitope which is a peptide sequence that comprises an MHC class II binding motif and can be suggested on the surface of an antigen-presenting cell by an MHC class II molecule. The peptide antigen of the present application may also contain a CD8+ T cell epitope which is a peptide sequence that comprises an MHC class I binding motif and can be suggested on the cell surface by an MHC class I molecule. The peptide antigen of the present application may also contain both of the CD4+ T cell epitope and CD8+ T cell epitope.

The term, "MHC (major histocompatibility complex)" or "major histocompatibility complex" is a protein which plays a role of providing an antigen piece into an immunocyte so that the immunocyte can distinguish self and non-self molecules, and there are two kinds of MHC class I and MHC class II, and MHC class I is present in all cells having nuclei, whereas MHC class II is discovered in an antigen-presenting cell. The MHC class I molecule interacts with a CD8+ cytotoxic T cell and plays an important role of destroying a rejection reaction of organ transplantation or infected cells. The MHC class II molecule plays an important role of recognizing a non-self antigen through interaction with a CD4+ T-helper cell and causing cell-mediated immunity.

In general, in an adaptive immune response, when an antigen enters the body, an antigen-presenting cell can ingest it and decompose it into short peptide fragments, and the peptide can bind to an MHC class I or MHC class II molecule in the cell and be delivered on the cell surface. As such, when the peptide of the antigen binds to the MHC class I or MHC class II and is presented on the cell surface of the antigen-presenting cell, a T cell recognizes it through a T cell receptor (TCR) and is activated, and initiates an immune response. In this aspect, the peptide antigen of the present application may correspond to an epitope of the T cell.

In other preferable embodiment, the peptide antigen may be a peptide derived from a tumor antigen, for example, tumor-associated antigen (TAA), a tumor-specific antigen (TSA) or a tumor-derived neoantigen; an antigen of an infection source, for example, an antigen derived from a virus, a bacterium, a parasite, or a fungus; an autoantigen known or suspected to induce autoimmunity; or an allergy-inducing antigen (allergen) known or suspected to induce allergy, but not limited thereto.

For example, the peptide antigen may comprise a part of a tumor antigen, an antigen of an infection source, an autoantigen, or an allergy-inducing antigen, in silico predicted or known to bind to an MHC class I or MHC class II molecule. Such antigens may be a CD8+ or CD4+ T cell epitope, but not limited thereto.

The peptide antigen may comprise a natural or non-natural amino acid sequence, an amino acid modified after translation, or a peptide mimic which can induce an immune response, for example, a T cell or B cell response, in a subject. For example, the peptide antigen may be about 5 to about 100, or about 5 to about 50 amino acids. For example, the peptide antigen may be about 7 to 35 amino acids, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids, but not limited thereto.

The term "tumor antigen" may be interchangeably used with "cancer antigen" , and refers to a molecule causing an immune response as an antigen expressed in a tumor (cancer). This immune response may accompany antibody production, or activation of a specific immune-competent cell, or both of them.

The tumor antigen may be derived from an organism having a tumor, a whole tumor cell killed or inactivated, or a lysate, and includes any antigen derived from a tumor. The lysate is a substance generated from application of a process of causing division of a normal structure of a cell. In addition, the tumor antigen includes any protein or other substances with antigen characteristics, which are included in a tumor cell and is expressed differently from a normal cell.

For example, the tumor antigen may include a tumor-associated antigen (TAA), a tumor-specific antigen (TSA) or a tumor-derived neoantigen.

The tumor-associated antigen (TAA) is an antigen which appears more frequently in cancer cells than normal cells, or is shown in other differentiation step than in normal cells, and is a tumor shared antigen which is present in trace amounts in normal cells. Accordingly, there is a high possibility that an immune response using it will be nullified by auto-tolerance, which is an immunosuppressive mechanism to prevent damage to self-cells, or conversely, an undesired organ may be attacked due to autoimmunity.

The example of the tumor-associated antigen (TAA), may include CT (Cancer-testis) antigen, EGFR, M12, M20, M21, M30, M44, Ova, Melan-A, PSMA(Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E) [for example, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2(MAGE-B2), MAGE-Xp3(MAGE-B3), MAGE-Xp4(MAGE-B4), MAGE-C1, MAGE-C2, MAGE-C3, MAGE-C4, MAGE-C5, etc.], GAGE (G antigen) [for example, GAGE-1, GAGE-2, GAGE-3, GAGE-4, GAGE-5, GAGE-6, GAGE-7, GAGE-8, GAGE-9, etc.], BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, N-RAS, K-RAS, RCAS1, $\alpha$-fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA, but not limited thereto.

The tumor-specific antigen (TSA) refers to an antigen that is specifically present only in cancer cells. In particular, when a tumor proliferates in a cancer patient, a new antigen epitope capable of stimulating a T cell as a gene mutation specific to a cancer cell is generated, and this is called a neoantigen. In other words, the neoantigen includes a gene mutation specific to a cancer cell, and is selectively expressed only in a cancer cell differently from a tumor shared antigen expressed in a trace amount also in a normal cell, so it is recognized as a non-self foreign epitope by an autoimmune system to cause a strong anticancer immune activity.

When a peptide generated from DNA in which mutation occurs is displayed on the MHC on the cell surface, a T cell receptor (TCR) recognizes it, and a mutation is not generated in a normal cell or tissue, so a neoantigen-specific T cell is free from auto-tolerance or autoimmune problems. Due to this advantage, the neoantigen is considered an ideal target for T cell-based cancer immunotherapy.

Causes generating the neoantigen include frame-shift deletion or insertion, where decoding of a genetic code is misaligned, as one or more nucleotides consisting of DNA are added or deleted, point mutation occurring as one nucleotide is substituted with another, other missense mutations, splice-site mutation, read-through mutation or gene-fusion mutation, and the like, but not limited thereto.

The neoantigen is predicted through specific cancer cell genome analysis of an individual cancer patient, and as an example, it may extract DNA and then analyze base sequences by obtaining cancer cells in a tumor of a patient, and compare and analyze them with base sequences of normal cells to select parts in which mutation occurs, and then identify a neoantigen which stimulates a T cell among base sequences of various parts in which mutation occurs. Herein, processing big data such as next gene sequencing (NGS), whole-exome sequencing (WES), or RNA sequencing. a computer program for predicting MHC binding, or an artificial intelligence (AI) for predicting a neoantigen, or the like may be utilized, but not limited thereto. As a mutation form is not shared between patients, so the neoantigen can be prepared as a personalized cancer vaccine.

As one specific example, the tumor antigen may be represented by any one amino acid sequence of SEQ ID NOs: 1 to 44, but not limited thereto.

The antigen of the infection source may be an antigen derived from a virus, a bacterium, a parasite, or a fungus.

For example, the virus-derived antigen may be an antigen derived from Varicella virus, Smallpox virus, Ebola virus, Marburg virus, Dengue virus, influenza virus, parainfluenza virus, respiratory syncytial virus, Measles virus, human immunodeficiency virus, human papillomavirus, varicella-zoster virus, Herpes simplex virus, cytomegalovirus, Epstein-Barr virus, JC virus, rhabdovirus, rotavirus, rhinovirus, adenovirus, papillomavirus, parvovirus, picornavirus, poliovirus, mumps inducing virus, rabies inducing virus, reovirus, rubella virus, togavirus, orthomyxovirus, retrovirus, hepadnavirus, coxsackievirus, equine encephalitis virus, Japanese encephalitis virus, yellow fever virus, Rift Valley fever virus, hepatitis A virus, hepatitis B virus, hepatitis C virus, hepatitis D virus or hepatitis E virus, but not limited thereto.

The bacteria-derived antigen may be an antigen derived from Borrelia species, Bacillus anthracis, burgdorferi, Bordetella pertussis, Camphylobacter jejuni, Chlamydia species, Chlamydial psittaci, Chlamydial trachomatis, Clostridium species, Clostridium tetani, Clostridium botulinum, Clostridium perfringens, Corynebacterium diphtheriae, Coxiella

EP 4 563 591 A1

species, Enterococcus species, Erlichia species, Escherichia coli, Francisella tularensis, Haemophilus species, Haemophilus influenzae, Haemophilus parainfluenzae, Lactobacillus species, Legionella species, Legionella pneumophila, Leptospirosis interrogans, Listeria species, Listeria monocytogenes, Mycobacterium species, Mycobacterium tuberculosis, Mycobacterium leprae, Mycoplasma species, Mycoplasma pneumoniae, Neisseria species, Neisseria meningitidis, Neisseria gonorrhoeae, Pneumococcus species, Pseudomonas species, Pseudomonas aeruginosa, Salmonella species, Salmonella typhi, Salmonella enterica, Rickettsia species, Rickettsia ricketsii, Rickettsia typhi, Shigella species, Staphylococcus species, Staphylococcus aureus, Streptococcus species, Streptococccus pnuemoniae, Streptococcus pyrogenes, Streptococcus mutans, Treponema species, Treponema pallidum, Vibrio species, Vibrio cholerae, or Yersinia pestis, but not limited thereto.

The fungus-derived antigen may be a fungus-derived antigen selected from Candida species, Cryptococcus species, Coccidioides species, Histoplasma species and Aspergillus species, but not limited thereto.

The parasite-derived antigen may be an antigen derived from Plasmodium, Trypanosome, Schistosome or Leishmania, but not limited thereto.

The term "carrier protein" refers to a protein which is linked to a peptide antigen and improves recombinant expression of the peptide antigen (for example, high expression rate, consistent expression rate, etc.), improves purification, enhances physical properties, stabilizes, or increases immunogenicity, and/or improves delivery into an immunoresponsive cell. In the present application, the carrier proteins may be linked to the N-terminus and C-terminus of the peptide antigen, respectively, and in order to distinguish them, each of them refers to a first carrier and a second carrier, respectively.

At least one of the carrier proteins may be linked to the N-terminus, C-terminus, or both, respectively. For example, 1, 2, or 3, or more carrier proteins may be linked to the N-terminus, C-terminus, or both, respectively.

The first carrier protein and the second carrier protein are same or different, and may be at least one of NDPK (nucleoside diphosphate kinase B), CSTA(Cystatin-A) [for example, human CSTA (hCSTA) or mouse CSTA (mCSTA), etc.], Trx(Thioredoxin) [for example, human Trx (hTRx) or mouse TRx (mTRx), etc.], RPL7Am (50S ribosomal protein L7Ae), Samp2a (Small archaeal modifier protein 2), TE (Tenascin) [for example, TE1, TE2, TE3.1, TE3.2, TE4, etc.], TM1112 (Thermotoga maritima Cupin_3 domain-containing protein), TrxA (Thioredoxin 1), TTrx (Thermosipho africanus Thioredoxin), or PSBD (peripheral subunit-binding domain) or fragments thereof, but not limited thereto. In addition, they may be codon-optimized for a host cell, or may be appropriately modified to comprise a mutation for some sequences or comprise an initiation methionine on the purpose of improvement of functional or structural stability. For example, the modification may be introduced by deleting, substituting or adding some of the sequence, for the purpose of removing a residue capable of involving in occurrence of dimerization or a redox reaction, inhibiting nucleic acid binding, inhibiting binding to Desampylase (UniProt: Q8U1Y4), reducing excessive binding affinity to human serum albumin, making quantification ease, inhibiting deamidation, and the like, but not limited thereto.

As a preferable embodiment, the first carrier protein located at the N-terminus can be selected in aspect of improving expression of an antigen, in consideration of a problem in that recombinant expression of an antigen may be difficult according to the sequence of a tumor antigen. For example, the first carrier protein may be selected on the purpose of expressing an antigen at a certain level or more, and/or expressing it consistently, and/or stabilizing it in a host expression system. For example, the first carrier protein may be at least one selected from TrxA, Trx (e.g., tTrx, mTrx, etc.), CSTA (e.g., tCSTA, mCSTA), TT-CSTA, TE, RPL7Am, Samp2a, TM1112, TT-TM1112, TT-Trx, but not limited thereto.

In addition, as a preferable embodiment, the second carrier protein located at the C-terminus, may be selected in an aspect of improving purification of an antigen, in consideration of a problem in that purification of an antigen (or removal of impurities) may be difficult depending on the sequence of a tumor antigen. For example, the second carrier protein may be selected on the purpose of improving purification by stabilizing the C-terminal structure of the fusion protein in a common purification system. For example, the second carrier protein may be at least one selected from NDPK, TrxA, CSTA (e.g., tCSTA, mCSTA), PSBD, PSBD-TT, but not limited thereto.

Furthermore, the carrier protein may include pan HLA DR binding epitope (PADRE), Tetanus toxin epitope (TT) or diphtheria toxoid or genetically detoxified variants thereof, Staphylococcus exotoxin or toxoid, or Pseudomonas aeruginosa exotoxin A or derivatives thereof, or the like, but not limited thereto.

As one specific example, the tumor antigen may be represented by any one amino acid sequence of SEQ ID NOs: 45 to 69, but not limited thereto.

The protein (for example, carrier protein and/or fusion protein) and/or polypeptide provided in the present description may be isolated and/or purified from nature, or recombinantly or chemically synthesized. When the amino acid sequence of the protein (for example, carrier protein and/or fusion protein) and/or polypeptide provided in the present description comprises methionine (Met, M) as the first amino acid residue, the protein or polypeptide may be recombinantly produced, and the methionine at the first amino acid location from the N-terminus may be encoded by an initiation codon. Therefore, when the amino acid sequence of the protein (for example, carrier protein and/or fusion protein) and/or polypeptide provided in the present description comprises methionine by recombinant production at the N-terminus, and when the protein or polypeptide is obtained by other methods (for example, chemical synthesis or isolation from nature) or is not located at the N-terminus in the fusion protein, it may be interpreted as comprising an amino acid sequence starting from the second amino acid residue except for methionine at the first location of the N-terminus by the recombinant production.

In one example of the fusion protein of the present application, an affinity tag may be linked to the N-terminus, C-terminus or both of them thereof.

The term "affinity tag" refers to a substance providing a site for attachment of a fusion protein for a specific substrate, when the fusion protein is purified in vitro, that is, experimentally. The affinity tag is a part of the fusion protein, and it should not induce immunogenicity or affect activity of the fusion protein.

The fusion protein of the present application, can be purified regardless of characteristics depending on the antigen sequence by comprising an affinity tag, and can produce protein at a superhigh speed as the fusion protein can be easily purified through affinity purification.

The affinity tag may be poly histidine (His), poly phenylalanine, poly alanine, streptavidin, maltose binding protein (MBP), intein, thioredoxin (Trx), protein A, NusA (N utilization substance A), β-galactosidase, or Glutathione-S-transferase (GST), but not limited thereto. For example, in case of the poly histidine tag, a peptide with a sequence in which 5 to 8 histidine are continuously arranged is used (for example, His-His-His-His-His-His), and the histidine tag has affinity for a divalent metal ion, so the fusion protein can be purified by affinity chromatography using Nickel immobilized bead, Cobalt immobilized bead, or the like.

The affinity tag may be cleaved or not cleaved under an extracellular or intracellular condition. Even if the affinity tag is not cleaved, they are not only safe in vivo, but also do not induce a structural change in the fusion protein, and do not substantially affect the fusion protein in exhibiting function thereof in a cell.

In one example of the fusion protein of the present application, a linker may be present between the peptide antigen and the first carrier protein, or a linker may be present between the peptide antigen and the second carrier protein, or both of them.

The term "linker" refers to a molecule or atom group which links or couples or binds two or more components. In the present application, each component of the fusion protein, for example, the peptide antigen and carrier protein, or carrier protein and affinity tag, may be linked or combined together through any appropriate means. The linker may have an additional function such as increasing or reducing water solubility, or increasing the distance between two components to be linked to give flexibility or increasing stability, or the like, but it is preferable not to induce immunogenicity or affect activity of the fusion protein.

The linker may be a peptide linker, and have a length of 1 to 10, or 2 to 10 amino acids, for example, 1, 2, 3, 4, 5, 6, 7, 8, 9, 10 or more (for example, 20 or less) amino acids, for example, a length of 11, 12, 13, 14, 15, 16, 17, 18, 19, 20 amino acids, but not limited thereto. As one example, the peptide linker may be composed of neutral amino acids (more specifically, Gly, Ser, Ala, Thr or a combination of these 4 amino acids). For example, it may be (GS)n, (G$_2$S)n, (G$_3$S)n, (G$_4$S)n, Gn, LE, SSGG or GGGGSGGGGG (herein, G is Gly, and S is Ser, and L is Leu, and E is Glu, and n is an integer of at least 1), and for example, it may be GS, GGGGS, LE, SSGG, GG, GGGGG or GGGGSGGGGG, but not limited thereto.

In one specific example, the linker may comprise a degradable peptide sequence cleavable by an intracellular enzyme, for example, protease, and cleavage of the linker may cause release of any component linked to the linker, for example, the affinity tag or carrier protein.

The term "linked" means that components are connected together directly or indirectly. Each component may be linked covalently or non-covalently. In addition, "linked" may mean maintaining chemical or physical binding of each component, after contacting to a cell, for example, an antigen-presenting cell or immunocyte and immunization. For example, when the antigen-presenting cell and immunocyte come into contact, components may be associated so that they do not freely disperse from one another. For example, two components are covalently linked to each other so that these two components can be prevented from dispersing or diffusing separately.

As one example, the size of the fusion protein of the present application is not particularly limited, but for example, it is advantageous that the size is small on the purpose to make removal of impurities ease in the purification process. In this aspect, the total size of the fusion protein may be a size of 500 kDa or less, 400 kDa or less, 300 kDa or less, 200 kDa or less, 100 kDa or less, 90 kDa or less, 80 kDa or less, 70 kDa or less, 60 kDa or less, 50 kDa or less, for example, 10 kDa to 100 kDa, 10 kDa to 90 kDa, 10 kDa to 80 kDa, 10 kDa to 70 kDa, 10 kDa to 60 kDa, 10 kDa to 50 kDa, 10 kDa to 40 kDa, 10 kDa to 30 kDa, but not limited thereto. Another example of the present application relates to a nucleic acid molecule encoding the fusion protein.

The term, "nucleic acid molecule" , "nucleic acid" , or "nucleic acid sequence" refers to a polymer of deoxyribonucleotide or ribonucleotide present in a single-strand or double-strand form. The nucleic acid molecule covers RNA genome sequences, cDNA and RNA sequences transcribed therefrom, and includes natural nucleic acid analogues unless otherwise mentioned particularly.

The nucleic acid molecule includes not only nucleic acid sequences encoding an amino acid sequence of the fusion protein, but also sequences complementary to the sequence. The complementary sequence includes not only completely complementary sequences, but also substantially complementary sequences, and this means for example, a sequence which can be hybridized with a nucleic acid sequence encoding the amino acid sequence of the fusion protein, under stringent conditions known in the art.

The fusion protein of the present application, is not limited thereto, but preferably, may be obtained by expression and purification by a recombinant method. Therefore, the present invention also provides an expression vector comprising a nucleic acid molecule encoding a fusion protein, and a cell transformed therewith, for expression and purification of the fusion protein.

Other example of the present application, relates to an expression vector comprising the nucleic acid molecule.

The term, "expression vector" refers to an operably linked nucleic acid structure in which a gene insert encoding a target protein is expressed. In one embodiment, the expression vector may be linear or circular, or single-strand or double-strand DNA, cDNA, RNA, or the like encoding 2 target proteins. The expression vector may form a part of a vector which can be used to transform, transduce or transfect a host, but not limited thereto, and may be transcribed and/or translated in vitro by itself.

The term, "operably linked" means that binding between nucleic acid sequences is functionally associated. For example, a coding sequence (e.g., sequence encoding a target protein) may be operably linked to appropriate regulatory elements so that reproduction, transcription and/or translation thereof is possible. For example, the coding sequence is operably linked to a promoter, when the promoter can promote transcription of the coding sequence. The regulatory elements need not to be adjacent to the coding sequence as long as they function properly. For example, an intervening sequence which is not translated but is transcribed may be present between a promoter sequence and a coding sequence, and the promoter sequence may still be considered as "operably linked" to the coding sequence.

Each element in the expression vector should be operably linked to each other, and the linkage of these element sequences may be performed by ligation (linkage) at a convenient restriction enzyme site, and when such a site is not present, it may be performed using a synthetic oligonucleotide adaptor or linker according to a common method.

The expression vector may comprise transcription and translation expression regulatory sequences which allow a corresponding gene to be expressed in a host. As the expression regulatory sequence, a promoter for conducting transcription, any operator sequence for regulating such transcription, and/or a sequence for regulating termination of transcription and translation can be comprised. The initiation codon and termination codon are generally considered a part of nucleic acid sequences encoding a target protein, and they should exhibit an action in a subject when a gene construct is administered and should be in frame with the coding sequence.

For example, the promoter means a DNA base sequence site to which transcription regulatory factors bind, and a promoter which can induce strong and stable gene expression may be used to increase a gene expression rate for the purpose.

The promoter may be constitutive or inductive. The promoter is not limited thereto, but illustrates early and late promoters of adenovirus, simian virus 40 (SV40) promoter, mouse mammary tumor virus (MMTV) promoter, HIV long terminal repeat (LTR) promoter, Moloney virus promoter, cytomegalovirus (CMV) promoter, Epstein virus (EBV) promoter, Rous sarcoma virus (RSV) promoter, RNA polymerase $\pm$ promoter, T3 and T7 promoters, main operators of phage lambda and promoter regions and the like may be exemplified.

In addition, the expression vector may appropriately comprise an adaptor or a linker, an enhancer, a selection marker (for example, antibiotic resistant marker), a replicable unit, a polyA sequence, a tag for purification, or other sequences of composition and induction known to regulate expression of a gene of a prokaryotic cell, or a eukaryotic cell or their viruses, and various combinations thereof and the like.

The expression vector may use various types of vectors such as a plasmid, a virus vector, a bacteriophage vector, a cosmid vector, and the like.

Other example of the present application, relates to a cell transformed with the expression vector.

In the present invention, for the transformed cell, as the host cell capable of stably and continuously cloning or expressing the expression vector, any host cell known in the art may be used, and the prokaryotic cell includes *E. coli,* for example, *E. coli* JM109, *E. coli* BL21, *E. coli* RR1, *E. coli* LE392, *E. coli* B, *E. coli* X 1776, *E. coli* W3110, Bacillus sp. strains such as Bacillus subtilis, Bacillus thuringiensis, and Enteric bacteria strains such as Salmonella typhimurium, Serratia marcescens, and various Pseudomonas species, and the like, and when transformed to an eukaryotic cell, as a host cell, yeasts *(Saccharomyces cerevisiae),* insect cells, plant cells, and animal cells, for example, CHO cell lines (Chinese hamster ovary), W138, BHK, COS-7, 293, HepG2, 3T3, RIN and MDCK cell lines, and the like may be used, but not limited thereto.

For introducing an expression vector into a cell, as known in the art, a suitable standard technology, for example, electroporation, electroinjection, microinjection, calcium phosphate co-precipitation, calcium chloride/rubidium chloride method, retroviral infection, DEAE-dextran, cationic liposome method, polyethylene glycol-mediated uptake, gene gun, and the like may be used, but not limited thereto. Then, a circular structure may be introduced in a linear form by cleaving with appropriate restriction enzyme.

A method for selecting the transformed cell may be easily conducted according to a method widely known in the art, using a phenotype expressed by a selection marker. For example, when the selection marker is a specific antibiotic resistant gene, by culturing a transformant in media containing the antibiotic, the transformant may be easily selected.

Culture of the transformed cells may be conducted by various methods known in the art. For example, the transformed cells were inoculated in culture media to conduct culture, and then at the point that the cell density reached a certain level, IPTG was added in media to induce protein expression and cultured, and thereby, proteins secreted in the cells or media may be obtained.

The proteins secreted in the cells or media may be obtained in purified form according to various purification methods known in the art, but preferably, it may be purified by affinity chromatography using an affinity tag. For example, proteins may be easily obtained using a resin column combined with glutathione, when the fusion protein is fused to GST, and IMAC (immobilized Metal Affinity Chromatography), when it is fused to His.

Other embodiment of the present application relates to a vaccine composition or immunogenic composition comprising the fusion protein, nucleic acid molecule, expression vector, or cell.

Other embodiment of the present application relates to a composition for prevention or treatment of cancer, infectious disease, autoimmune disease, or allergic disease, comprising the fusion protein, nucleic acid molecule, expression vector or cell.

The term, "immunogenic composition" means any composition capable of inducing an immune response. The term, "vaccine" refers to an immunogenic composition for reducing or preventing a risk of disease or infection, or improving or treating a conventional disease or infection by inducing an immune response.

These compositions may be formulations in a form which contains the fusion protein according to the present application and can be administered into a subject in order to cause an immune response. Therefore, the composition of the present invention may be conveniently used to prevent, improve or treat disease. After introduced into a subject or host, the composition may cause an immune response, and preferably, it may cause a T-cell mediated immune response.

The vaccine of the present invention may be an anticancer vaccine comprising a tumor antigen, for example, a tumor antigen sequence obtained from a patient's tumor analysis, particularly, a neoantigen sequence, and accordingly, proliferation and activation of a personalized anticancer T cell may be triggered at maximum to increase an anticancer immune effect.

The vaccine composition of the present application may also comprise an additional adjuvant which reinforces effectiveness of a vaccine. The appropriate adjuvant includes (1) aluminum salts (alum), for example, aluminum hydroxide, aluminum phosphate, aluminum sulfate, and the like; (2) oil-in-water emulsion formulations (containing or not containing a specific immunostimulant such as muramyl peptide or bacterial cell wall components), for example, (a) MF59 comprising 5% squalene, 0.5% tween 80 and 0.5% span 85 (selectively containing N-acetylmuramyl-L-alanyl-D-isoglutaminyl-L-alanine-2-(1',2'-dipalmitoyl-sn-glycero-3-hydroxyphosphoryloxy)-ethylamine (MTP-PE) in various amounts, which is not necessary), formulated as a particle with a micron or less (WO90/14837), (b) SAF comprising 10% squalene, 0.4% tween 80, 5% Pluronic-blocked polymer and N-acetyl-muramyl-L-threonyl-D-isoglutamine (thr-MDP), shaken to be microfluidized into a particle with a micron or less, or produce emulsion in a large particle size, and (c) RibiTM adjuvant system (RAS) comprising at least one bacterial cell wall component selected from the group consisting of monophosphoryllipid A (MPL), trehalose dimycolate (TDM) and cell wall skeleton (CWS), 2% squalene, and 0.2% tween 80; (3) saponin adjuvants; (4) complete Freund's adjuvants (CFA) and incomplete Freund's adjuvants (IFA); (5) cytokines, for example, interleukins (for example, IL-1, IL-2, IL-4, IL-5, IL-6, IL-7, IL-12, etc.), interferons (for example, gamma interferons), macrophage colony stimulating factors (M-CSF), tumor necrosis factors (TNF) and the like; (6) detoxified mutants of bacterial ADP-ribosylated toxins, for example, cholera toxins (CT), pertussis toxins (PT), or heat labile toxins (LT) of E. coli, particularly, LT-R72, CT-S109, PT-K9/G129 (WO93/13302 and WO92/19265); and other materials acting as an adjuvant which enhances effectiveness of the vaccine, but not limited thereto.

The compositions of the present application may further comprise a pharmaceutically acceptable carrier, diluent and/or excipient in a commonly used amount as necessary.

The pharmaceutically acceptable carrier includes lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxybenzoate, propylhydroxybenzoate, talc, magnesium stearate and mineral oils and the like, which are commonly used for formulation, but not limited thereto. The composition may further comprise a lubricant, a wetting agent, a sweetener, a flavoring agent, an emulsifier, a suspending agent, a preservative, and the like in addition to the above components.

The fusion protein and composition comprising the same as an active ingredient may be prepared in a unit dose form or prepared by inserting in a multidose container by formulating using a pharmaceutically acceptable carrier and/or excipient, according to a method which can be easily performed by those skilled in the art.

For example, the vaccine composition may comprise common salt water suspended or dissolved, or buffered aqueous solution media. For example, commonly, a diluent, for example, water, salt water, glycerol, ethanol and the like may be comprised, and an auxiliary substance, for example, a wetting agent, an emulsifier, a pH buffer, and the like may be present in the composition.

An appropriate for injection includes a sterilized aqueous solution (water soluble) or dispersion and sterilized powders for immediate preparation of a sterilized injection solution or dispersion. They should be stable under a preparation condition, and should be preserved against contamination of microorganisms such as bacteria or fungi. Contamination of microorganisms may be prevented using various antibacterial agents and antifungal agents, for example, paraben, chlorobutanol, phenol, sorbic acid, thimerosal and the like. In many cases, it is preferable to comprise an isotonic agent, for example, sugar or sodium chloride. Long-term absorption of the injection composition may be possible by using a drug which delays absorption, for example, aluminum monostearate or gelatin in the composition.

A sterilized injection solution is prepared by combining the fusion protein in a required amount and various other components listed above in the aforementioned solvent, and filtering and sterilizing other components other than the fusion protein and/or heat labile adjuvant, cytokine and the like, for example, buffer solution such as PBS, if necessary. In general, dispersion is prepared by comprising various sterilized active ingredients into a sterilized vehicle containing a basic dispersion medium and other components required from the aforementioned. In case of the sterilized powder for preparation of the sterilized injection solution, a preferable preparation method is vacuum drying and freeze-drying methods. By these methods, a powder of the active ingredient and any desired component is obtained from the aforementioned sterile-filtered solution thereof described above.

A patient subject to administration of the fusion protein or a composition comprising the same as an active ingredient may be a mammal, for example, a human, a primate including a monkey, etc., a rodent including a rat, a mouse, etc., and the like, but not limited thereto.

Other aspect of the present invention provides a method for causing, inducing or promoting an immune response against an antigen, comprising administering an effective dose of the fusion protein, vaccine composition and/or immunogenic composition into a patient.

The method for causing, inducing or promoting an immune response against an antigen may relate to a method for causing, inducing or promoting an immune response against an antigen in a cancer patient, and for example, comprises administering into a cancer patient.

The term, "immune response" is the result of directly or indirectly stimulating through cellular or cytokine mediation, and refers to a change in activity of a cell, for example, a B cell, a T cell or a monocyte, of an immune system. The immune response may be specific (T cell and/or B cell) and/or nonspecific immune response.

That the fusion protein is delivered according to the present invention, without limiting the action of the present invention in any way induces an immune response, and in particular, it is particularly useful for inducing a T cell response, for example, a CD4+ T cell response or a CD8+ T cell response against an antigen. The CD4+ T cell response and CD8+ T cell response may occur together or independently of a humoral response or other specific or nonspecific immune response.

Other aspect of the present invention is related to using the fusion protein of the present invention in relation to treatment and/or prevention of disease conditions. Examples of the disease which can be treated according to the present invention include various kinds of cancer diseases, infectious diseases, autoimmune diseases and allergic diseases.

For example, the cancer may be a solid cancer or a hematologic malignancy, and as a non-limitative example, it may be breast cancer, lung cancer, prostate cancer, ovarian cancer, brain cancer, liver cancer, cervical cancer, endometrial cancer, uterine cancer, colon cancer, colorectal cancer, rectal cancer, kidney cancer, nephroblastoma, skin cancer, oral squamous carcinoma, epidermoid carcinoma, nasopharyngeal carcinoma, head and neck cancer, bone cancer, esophageal cancer, bladder cancer, lymphatic cancer (for example, Hodgkin' s lymphoma, or non-Hodgkin' s lymphoma), stomach cancer, pancreatic cancer, testis cancer, thyroid cancer, follicular carcinoma, melanoma, myeloma, multiple myeloma, mesothelioma, osteosarcoma, myelodysplastic syndrome, mesenchymal origin tumor, soft tissue sarcoma, liposarcoma, gastrointestinal stromal sarcoma, malignant peripheral nerve sheath tumor (MPNST), Ewing sarcoma, leimyosarcoma, mesenchymal chondrosarcoma, lymphosarcoma, fibrosarcoma, rhabdomyosarcoma, teratocarcinoma, neuroblastoma, medulloblastoma, glioma, skin benign tumor, or leukemia. The lung cancer may be for example, small cell lung cancer (SCLC) or non-small cell lung cancer (NSCLC). The leukemia may be for example, acute myeloid leukemia (AML), chronic myeloid leukemia (CML), acute lymphocytic leukemia (ALL), or chronic lymphocytic leukemia (CLL). The subject to be treated may be a subject receiving secondary anti-hyperproliferative therapy. For example, the secondary anti-hyperproliferative therapy may be chemotherapy, radiotherapy, immunotherapy, phototherapy, cryotherapy, toxitherapy, hormone therapy, or surgery.

Therefore, other aspect of the present invention provides a method for preventing, improving and/or treating cancer, infectious disease, autoimmune disease, or allergic disease, comprising administering an effective dose of the fusion protein, vaccine composition and/or immunogenic composition into a patient. The method for preventing, improving and/or treating may be a method for preventing, improving and/or treating disease, for example, cancer, infectious disease, autoimmune disease, or allergic disease, by enhancing an immune response. Herein, the administering an effective dose of the fusion protein, vaccine composition and/or immunogenic composition into a patient, inhibits, halts, or delays occurrence or progression of disease conditions, or causes, induces, or otherwise promotes an immune response to be prevented.

Direct delivery of the composition may be generally systemic, subcutaneous, intradermal, intraperitoneal, endovascular (intravenous), intramuscular or local delivery, or is delivery through a gap of tissue. The composition may be also administered into a lesion. The administration therapy may be a single dose or multiple dose schedule.

The term, "effective dose" means an amount to achieve a desired result, when administered into a subject including humans, for example, an amount effective for treating or preventing cancer. The effective dose may vary depending on factors such as a subject's disease conditions, age, gender and body weight and the like. The dosage or treatment usage may be adjusted to provide an optimal treatment response as understood by those skilled in the art.

The treatment usage of a subject using a therapeutically effective dose may consist of a single administration, or as another example, may include a series of applications. The period of the treatment time is determined according to various factors such as the severity of disease, age of the subject, concentration of the vaccine, the reactivity of the patient to the vaccine or combinations thereof. In addition, it will be understood that the effective dosage of the vaccine used for treatment may be increased or decreased during the course of individual treatment usage. A change in dosage may occur, and it may be clarified through standard diagnostic analysis known in the art. The vaccine of the present invention, for example, the cancer vaccine may be administered, before, during or after treatment using a common anticancer agent, radiotherapy, hormone therapy, biotherapy and/or surgical tumor excision.

[ADVANTAGEOUS EFFECTS]

The present invention relates to a fusion protein, comprising a peptide antigen containing a T cell epitope, and a carrier protein linked to the N-terminus or C-terminus of the peptide antigen, or both of them; a nucleic acid molecule encoding the fusion protein; an expression vector comprising the nucleic acid molecule; a cell transformed with the expression vector; and an immunogenic composition comprising the fusion protein, nucleic acid molecule, expression vector or cell, and has an effect of improving expression, physical properties, stability and/or immunogenicity of an antigen, and improving delivery of an antigen into an immune response cell.

[BRIEF DESCRIPTION OF THE DRAWINGS]

FIG. 1 is the SDS-PAGE result of confirming preparation of the fusion protein prepared according to one example of the present application.
FIG. 2 is the purity test (SE-HPLC) of the fusion protein prepared according to one example.
FIG. 3 is the result which shows the endotoxin content according to size exclusion chromatography fractions of the fusion protein prepared according to one example of the present application.
FIG. 4 is the result which shows the tumor inhibitory effect when the anticancer vaccine prepared according to one example of the present application is administered into the MC38 mouse model.
FIG. 5 is the result which shows the tumor inhibitory effect when the anticancer vaccine prepared according to one example of the present application is administered into the CT26 mouse model.
FIG. 6 shows the TIL analysis result according to administration of the anticancer vaccine prepared according to one example of the present application into the MC38 mouse model.
FIG. 7 shows the TIL analysis result according to administration of the anticancer vaccine prepared according to one example of the present application into the CT26 mouse model.
FIG. 8 shows the TIL analysis result according to administration of the anticancer vaccine prepared according to one example of the present application into the B16-F10 mouse model.
FIG. 9 shows the structure of the fusion protein according to one example of the present application.

[MODE FOR INVENTION]

Hereinafter, the present invention will be described in more detail by the following examples. However, they are intended to illustrate the present invention only, but the scope of the present invention is not limited by these examples.

**Example 1. Selection of epitopes**

Whole exome sequencing (WES) of cancer cells and normal cells, for identification of mutation in a mouse and selection of an epitope binding to MHC, and mRNA sequencing for identifying expression of mutant genes, were performed. By analyzing the WES result, mutations in which protein sequences are changed (missense mutation, frameshift mutation, insertion-deletion) and the MHC type of the mouse were identified.

Specifically, as the normal cells, tail tissues of mice were used, and in case of the cancer cells, a mouse melanoma cell line, B16-F10 and mouse colorectal cancer cell lines, MC38 and CT26 were used to perform sequencing (NGS). NGS was performed in Macrogen, and WES analyzed missense Single-nucleotide polymorphism (SNP) mutation with Mutect2 algorithm. Based on the SNP, a 13mer sequence was added to find a 27mer amino acid.

In mRNA sequencing, only genes with an expression level, that is, RPKM (Reads Per Kilobase Million) value of 10 or higher, were selected from the WES sequencing result.

The epitope sequences obtained from the result were inputted in a published program of NetMHCCons and NetMHCPanII. For the MHC type, a phenotype appropriate for a mouse was inputted, and Strong Binding (SB) and Weak Binding (WB) criteria were set to default. NetMHCCons was analyzed by 9mer, and NetMHCPanII was analyzed by 13mer. Scores were imputed by inputting in another program, IEBD Immunogenicity program, and analyzing by 9mer.

$$\text{Predicted Score} = 10 \times \text{number of NetMHCCons Binders}$$

$$+ \text{ number of NetMHCPanII Binders}$$

$$+ \text{ IEDB Immunogenicity Score}$$

Considering that the higher the predicted score, the greater the probability of showing drug efficacy, all immune responses were confirmed by selecting the top 20, and among them, evaluation of anticancer efficacy was conducted by selecting a substance showing an immune response or a substance which was easy to prepare. In addition, using these selected epitopes, fusion proteins for anticancer vaccines were prepared.

[Table 1]

| Epitopes (In the table below, "CT+number" is an arbitrarily named Neoantigen number of the CT26 colorectal cancer cell line, and "M + number" is an arbitrarily named Neoantigen number of the Melanoma cell line (B16-F10), and "OVA" means an antigen of Ovalbumin, and Cpne1, Irgq, Aatf represent Neoantigens of the MC38 colorectal cancer cell line) | | | |
|---|---|---|---|
| Number | Epitope name | Amino acid sequence | SEQ ID NO: |
| 1 | CT1 | RNNSYTSYIMAICGMPL | 1 |
| 2 | CT2 | CWKYLSVQSQLFRGSSL | 2 |
| 3 | CT3 | GGISVKEHIEVNVVPLT | 3 |
| 4 | CT20 | MANQVIRCTAAVAWEAG | 4 |
| 5 | CT24 | PLEAMYMQWPVIAVNNG | 5 |
| 6 | CT25 | LHLEETLAGFWARLLER | 6 |
| 7 | CT4 | GGISVKEHIEVNVVPLT | 7 |
| 8 | CT5 | IYLESVAIMPQLFMVSK | 8 |
| 9 | CT6 | PLEAMYMQWPVIAVNNG | 9 |
| 10 | EGFR+12mer | TSTVQLIMQLMPFGCLLDYVREH | 10 |
| 11 | EGFR+4mer | QLIMQLMPFGCLLDY | 11 |
| 12 | EGFR+8mer | TVQLIMQLMPFGCLLDYVR | 12 |
| 13 | EGFRwt | IMQLMPFGCLL | 13 |
| 14 | M12 | TPPPEEAMPFEFNGPAQGDHSQPPLQV | 14 |
| 15 | M20 | FRRKAFLHWYTGEAMDEMEFTEAESNM | 15 |
| 16 | M21 | SSPDEVALVEGVQSLGFTYLRLKDNYM | 16 |
| 17 | M22 | PKPDFSQLQRNILPSNPRVIRFHINWD | 17 |
| 18 | M30 | PSKPSFQEFVDWENVSPELNSTDQPFL | 18 |
| 19 | M30.2 | DWENVSPELNSTDQP | 19 |
| 20 | M30.3 | FVDWENVSPELNSTDQPFL | 20 |
| 21 | M30.4 | QEFVDWENVSPELNSTDQPFLPS | 21 |

(continued)

| Number | Epitope name | Amino acid sequence | SEQ ID NO: |
|--------|--------------|---------------------|------------|
| | | Epitopes (In the table below, "CT+number" is an arbitrarily named Neoantigen number of the CT26 colorectal cancer cell line, and "M + number" is an arbitrarily named Neoantigen number of the Melanoma cell line (B16-F10), and "OVA" means an antigen of Ovalbumin, and Cpne1, Irgq, Aatf represent Neoantigens of the MC38 colorectal cancer cell line) | |
| 22 | M30.5 | SFQEFVDWENVSPELNSTDQPFLPSAP | 22 |
| 23 | M44 | EFKHIKAFDRTFANNPGPMVVFATPGM | 23 |
| 24 | melanA_short | HGHSYTTAEELAGIGILTV | 24 |
| 25 | MelanA_long | YPKKGHGHSYTTAEELAGIGILTVILG | 25 |
| 26 | melanA+4mer | HGHSYTTAEELAGIGILTVILGV | 26 |
| 27 | OVA27 | DEVSGLEQLESIINFEKLTEWTSSNVM | 27 |
| 28 | OVA27.2(DKVS) | DKVSGLEQLESIINFEKLTEWTSSNVM | 28 |
| 29 | OVA27.3(DRVS) | DRVSGLEQLESIINFEKLTEWTSSNVM | 29 |
| 30 | OVA27.4(DRVK) | DRVKGLEQLESIINFEKLTEWTSSNVM | 30 |
| 31 | OVA8.1 | SIINFEKL | 31 |
| 32 | OVA8.2 | LESIINFEKLTE | 32 |
| 33 | OVA8.3 | EQLESIINFEKLTEWT | 33 |
| 34 | OVA8.4 | GLEQLESIINFEKLTEWTSS | 34 |
| 35 | PSMA+12mer | LCAGALVLAGGFFLLGFLFGW | 35 |
| 36 | PSMA+12mer_C2S | LSAGALVLAGGFFLLGFLFGW | 36 |
| 37 | PSMA+4mer | ALVLAGGFFLLGF | 37 |
| 38 | PSMA+8mer | AGALVLAGGFFLLGFLF | 38 |
| 39 | PSMAwt | VLAGGFFLL | 39 |
| 40 | Survivin | KKQFEELMLGEFLKLDRERAKNKIAKE | 40 |
| 41 | Cpne1 | GSNGDPSSPYSLHYLSPTGVNEYLTAL | 41 |
| 42 | Irgq | PGDSQNAAKARDETAALLNSAVLGAAP | 42 |
| 43 | Aatf | HVLSKLLSFMAPIDHTTMSDDARTELF | 43 |
| 44 | AFP | EAYEEDRETFMNKFIYEIARRHPFLYA | 44 |

## Example 2. Selection of carrier proteins

### 2-1. Design of carrier proteins

Neoantigens induce T-cell immunity by binding to MHC-I (Major Histocompatibility Complex-I) or MHC-II protein, and when Synthetic Long Peptide (SLP) comprising a neoantigen is used as an antigen, it has been known that a half-life in a body is generally short, and it has been known that the solubility is low depending on the sequence. In order to supplement such a disadvantage of SLP, an attempt was made to prepare of a fusion protein of a neoantigen and a carrier protein, and the fusion protein was expressed in E. coli and was tried to be produced through a purification process.

The neoantigen was expressed with an ORF (Open Reading Frame) consisting of (CP1) - (linker 1) - (neoantigen) - (linker 2) - (CP2) - (linker 3) - (His-tag). Since the neoantigens are different for each patient, in order to consistently express them in E. coli above a certain level, CP1 which is expressed well in E. coli was arranged at the ORF N-terminus. In addition, as there may be a problem of difficult purification depending on the neoantigen sequence, structurally stable CP2 was arranged at the ORF C-terminus. Furthermore, as physiochemical properties of the sequences of the expressed antigens are different, an affinity tag, His-tag was arranged at the ORF C-terminus to purify antigens regardless of the antigen sequence characteristics. CP2 was arranged between the antigen and His-Tag to prevent any antigen sequence from interrupting purification by the affinity tag, and the main elements of the ORF were linked with a linker consisting of glycine and serine. The kinds of carrier proteins used in the present invention are as follows.

[Table 2]

| Carrier proteins | | |
|---|---|---|
| SEQ ID NO: | Carrier protein | Amino acid sequence |
| 45 | human CSTA (Cystatin-A) | MIPGGLSEAKPATPEIQEIVDKVKPQLEEKTNETYGKLEAVQYKTQVVAGTNYYI KVRAGDNKYMHLKVFKSLPGQNEDLVLTGYQVDKNKDDELTGF |
| 46 | human CSTA(codon optimized) | |
| 47 | hTrx.v1 (human Thioredoxin) | MVKQIESKTAFQEALDAAGDKLVVVDFSATWCGPCKMIKPFFHSLSEKYSNVIFL EVDVDDSQDVASESEVKSMPTFQFFKKGQKVGEFSGANKEKLEATINELV<br>- Mutant (C62S, C69S, C73S) |
| 48 | hTrx.v2 (human Thioredoxin) | MVKQIESKTAFQEALDAAGDKLVVVDFSATWCGPCKMIKPFFHSLSEKYSNVIFL EVDVDDCQDVASECEVKSMPTFQFFKKGQKVGEFSGANKEKLEATINELV<br>- Mutant (C73S) |
| 49 | hTrx.v3 (human Thioredoxin) | MVKQIESKTAFQEALDAAGDKLVVVDFSATWSGPSKMIKPFFHSLSEKYSNVIFL EVDVDDSQDVASESEVKSMPTFQFFKKGQKVGEFSGANKEKLEATINELV<br>- Mutant (C32S, C35S, C62S, C69S, C73S) |
| 50 | hTrx.wt (human Thioredoxin) | MVKQIESKTAFQEALDAAGDKLVVVDFSATWCGPCKMIKPFFHSLSEKYSNVIFL EVDVDDCQDVASECEVKCMPTFQFFKKGQKVGEFSGANKEKLEATINELV |
| 51 | mCSTA.midGGGGG (mouse Cyst at in-A) | MIPGGLTEARPATAEVQEIADRVKAQLEEETNEKYEIFKAVQYKTQVGGGGGVNY FIKMDVGGGCFTHIKVFKDLSGKNNLELTGYQTNKTEDDELTYF<br>-Mutant (GGGGG substitution) |
| 52 | mCSTA (mouse Cystatin-A) | MIPGGLTEARPATAEVQEIADRVKAQLEEETNEKYEIFKAVQYKTQVVAGVNYFI KMDVGGGCFTHIKVFKDLSGKNNLELTGYQTNKTEDDELTYF |
| 53 | RPL7Am (50S ribosomal protein L7Ae) | MAVYVKFKVPEEIQKELLDAVAKAQKIKKAANEVTKAVERGIAKLVIIAEDVKPE ELVAHLPYLCEEKGIPYAYVASKQDLGKAAGLEVAASSVAIINEGDAEELKVLIE KVNVLKQ<br>- Mutant (G30A, V57L) |
| 54 | Samp2a (Small archaeal modifier protein 2) | MKMIKVKVVGRNIEKEIEWREGMKVRDILRAVGFNTESAVAKVNGKVVLEDDEVK DGDFVEVIPVWT<br>- Mutant (I9V, I40V, V66W, S67T) |
| 55 | TE1 (Human Tenascin 1) | MPPKDLVVTEVTEETVNLAWDNEMRVTEYLVVYTPTHEGGLEMQFRVPGDQTSTI IQELEPGVEYFIRVFAILENKKSIPVSARVATYLPAP<br>(Tenascin 625-715 (UniProt P24821); when expressed at the N-terminal of the epitope, M (methionine) is inserted) |
| 56 | TE2 (Human Tenascin 1) | MEGLKFKSIKETSVEVEWDPLDIAFETWEIIFRNMNKEDEGEITKSLRRPETSYR QTGLAPGQEYEISLHIVKNNTRGPGLKRVTTTRLD<br>(Tenascin 716-804; when expressed at the N-terminal of the epitope, M (methionine) is inserted) |
| 57 | TE3.1-TE4 | MAPSQIEVKDVTDTTALITWFKPLAEIDGIELTYGIKDVPGDRTTIDLTEDENQY |

(continued)

| | SEQ ID NO: | Carrier protein | Amino acid sequence |
|---|---|---|---|
| Carrier proteins | | | |
| | | (Human Tenascin 1) | SIGNLKPDTEYEVSLISRRGDMSSNPAKETFTTGLDAPRNLRRVSQTDNSITLEW RNGKAAIDSYRIKYAPISGGDHAEVDVPKSQQATTKTTLTGLRPGTEYGIGVSAV KEDKESNPATINAATELDTPKD<br>(Tenascin 805-990; when expressed at the N-terminal of the epitope, M (methionine) is inserted) |
| | 58 | TE3.1 (Human Tenascin 1) | MAPSQIEVKDVTDTTALITWFKPLAEIDGIELTYGIKDVPGDRTTIDLTEDENQY SIGNLKPDTEYEVSLISRRGDMSSNPAKETFTTGLD<br>(Tenascin 805-894; when expressed at the N-terminal of the epitope, M (methionine) is inserted) |
| | 59 | TE3.2-TE4 (Human Tenascin 1) | MLDAPSQIEVKDVTDTTALITWFKPLAEIDGIELTYGIKDVPGDRTTIDLTEDEN QYSIGNLKPDTEYEVSLISRRGDMSSNPAKETFTTGLDAPRNLRRVSQTDNSITL EWRNGKAAIDSYRIKYAPISGGDHAEVDVPKSQQATTKTTLTGLRPGTEYGIGVS AVKEDKESNPATINAATELDTPKD<br>(Tenascin 803-990; when expressed at the N-terminal of the epitope, M (methionine) is inserted) |
| | 60 | TE3.2 (Human Tenascin 1) | MLDAPSQIEVKDVTDTTALITWFKPLAEIDGIELTYGIKDVPGDRTTIDLTEDEN QYSIGNLKPDTEYEVSLISRRGDMSSNPAKETFTTGL<br>(Tenascin 803-893; when expressed at the N-terminal of the epitope, M (methionine) is inserted) |
| | 61 | TE4 (Human Tenascin 1) | MAPRNLRRVSQTDNSITLEWRNGKAAIDSYRIKYAPISGGDHAEVDVPKSQQATT KTTLTGLRPGTEYGIGVSAVKEDKESNPATINAATELDTPKD<br>(Tenascin 895-990; when expressed at the N-terminal of the epitope, M (methionine) is inserted) |
| | 62 | TM1112 (Thermotoga maritima Cupin_3 domain-containing protein) | MEVKIEKPTPEKLKELSVEKWPIWEKEVSEFDWYYDTNRTAYILEGKVEVTTEDG KKYVIEKGDLVTFPKGLRSRWKVLEPVRMHYNLF<br>-Mutant (E39R, C41A, C74S, K84M) |
| | 63 | TrxA.v2 (Escherichia coli, Thioredoxin 1) | MSDKIIHLTDDSFDTDVLKADGAILVDFWAEWSGPSKMIAPILDEIADEYQGKLT VAKLNIDQNPGTAPKYGIRGIPTLLLFKNGEVAATKVGALSKGQLKEFLDANLA<br>- TrxA mutant (C33S, C36S) |
| | 64 | TrxA (Escherichia coli, Thioredoxin 1) | MSDKIIHLTDDSFDTDVLKADGAILVDFWAEWCGPCKMIAPILDEIADEYQGKLT VAKLNIDQNPGTAPKYGIRGIPTLLLFKNGEVAATKVGALSKGQLKEFLDANLA |
| | 65 | TTrx (Thermosipho africanus Thioredoxin) | MKIEYFKNDKSSVSKAMLPKIQTIAKNFDIDIEVIDVIENPSYPAQKLVFTVPTV IILDKEFEIKRFARNFSISEVINTIERYLEISNKE<br>-Mutant (C11S, C14S) |
| | 66 | PSBD (peripheral subunit-binding domain) | VIAMPSVRKYAREKGVDIRLVQGTGKNGRVLKEDIOAWLA - Mutant (F166W) |
| | 67 | PSBD_G | VIAMPSVRKYAREKGVDIRLVQGTGKGGRVLKEDIDAWLA - Mutant (N155G, F166W) |

(continued)

| Carrier proteins | | |
|---|---|---|
| SEQ ID NO: | Carrier protein | Amino acid sequence |
| 68 | NDPK (nucleoside di-phosphate kinase B [Mus musculus]) | MANLERTFIAIKPDGVQRGLVGEIIKRFEQKGFRLVAMKFLRASEEHLKQHYIDL KDRPFFPGLVKYMNSGPVVAMVWEGLNVVKTGRVMLGETNPADSKPGTIRGDFCI QVGRNIIHGSDSVESAEKEIHLWFKPEELIDYKSCAHDWVYE<br>-wild type |
| 69 | TT (Tetanus Toxoid anti-gen) | QYIKANSKFIGITEL<br>- Tetanus Toxin (830-844) peptidic epitope : as part of Tetanus toxin from Clostridium tetani |

## 2-2. Result of preparing of neoantigen fusion protein by carrier protein

Since protein sequences of neoantigens in anticancer vaccines are different from each other, even if fused with a carrier protein, pI and hydrophobicity are different depending on the neoantigen protein sequence, so it is difficulty in purifying all antigens with ion exchange chromatography or hydrophobic interaction chromatography. For this reason, antigen proteins were to be purified using affinity, and purification was performed by selecting His-tag, which has a small tag size of 6 histidine amino acids and a high protein binding capacity of 20 mg per 1 mL of Resin as an affinity tag. In addition, in order to make and compare the structure in which the neoantigen is arranged at the N-terminus, an MBP tag was introduced at the N-terminus and expressed and purified, and then cut with TEV protease to prepare the antigen. Antigens primarily purified with the affinity tag were polished using secondary size-exclusion chromatography (SEC). The method of cloning, culturing and purification were the same as Example 3. The result of preparing of the neoantigen fusion proteins combined with each carrier protein was as follows.

[Table 3]

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| hCSTA | - | M12 | pc0726 | hCSTA-M12-His | 3.1 | 29.8 |
| | - | M21 | pc0725 | hCSTA-M21-His | 3.41 | 6.2 |
| | - | M30 | pc0721 | hCSTA-M30-His | 3.78 | 79.4 |
| | - | M44 | pc0722 | hCSTA-M44-His | 3.04 | 21.6 |
| | - | MelanA | pc0638 | CSTA-GGGGS-melanAOmer-SS-GG-His | 4.55 | 14.1 |
| | - | OVA27 | pc0637-P01 | CSTA-GGGGS-OVA27-SS-GG-His | 6.36 | 21.6 |
| | - | OVA27 | pc0637-P02 | CSTA-GGGGS-OVA27-SS-GG-His | 3 | 43.6 |
| | - | OVA27 | pc0637-P03 | CSTA-GGGGS-OVA27-SS-GG-His | 3.9 | 44.8 |
| | - | OVA27 | pc0666 | CSTA-GGGGS-OVA-GGGGS-GG-His | 9.54 | 49.6 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| | - | OVA27 | pc0695 | CSTA-GGGGS-OVA27-SS-GG-His | 2.79 | 27.9 |
| | - | OVA27.2 | pc0700 | CSTA-GGGGS-OVA27.2(DKVS)-SS-GG-His | 3.49 | 36.7 |
| | - | OVA27.3 | pc0701 | CSTA-GGGGS-OVA27.3(DRVS)-SS-GG-His | 3.4 | 35.7 |
| | - | OVA27.4 | pc0702 | CSTA-GGGGS-OVA27.4(DRVK)-SS-GG-His | 3.7 | 38.1 |
| hTrx.v1 | - | M30 | pc0764 | hTrx.v1-M30-His | 2.37 | 22.7 |
| | - | M44 | pc0761 | hTrx.v1-M44-His | 2.49 | 19.4 |
| | - | MelanA | pc0993 | hTrx.v1-MelanA-His | 0.733 | 3.5 |
| | PSBD | melanA | pc0995 | hTrx.v1-melanA-PSBD_G-His | 2.21 | 25.5 |
| hTrx.v3 | - | CT1 | pc0822 | hTrx.v3-CT1-His | 2.28 | 9.8 |
| | PSBD | CT1 | pc0868 | hTrx.v3-CT1-PSBD-His | 6.2 | 47.1 |
| | - | CT2 | pc0814 | hTrx.v3-CT2-His | 2.39 | 9.8 |
| | PSBD | CT2 | pc0882 | hTrx.v3-CT2-PSBD-His | 1.91 | 24.9 |
| | - | CT20 | pc0793 | hTrx.v3-CT20-His | 3.5 | 15.8 |
| | - | CT24 | pc0801 | hTrx.v3-CT24-His | 1.57 | 16.5 |
| | PSBD | CT24 | pc0888 | hTrx.v3-CT24-PSBD-His | 2.43 | 31.6 |
| | PSBD-TT | CT24 | pc0970 | hTrx.v3-CT24-PSBD-TT-His | 3.3 | 11.5 |
| | - | CT25 | pc0813 | hTrx.v3-CT25-His | 1.44 | 19.4 |
| | PSBD | CT25 | pc0889 | hTrx.v3-CT25-PSBD-His | 2.08 | 30.1 |
| | - | CT4 | pc0823 | hTrx.v3-CT4-His | 6.51 | 91.2 |
| | PSBD | CT4 | pc0885 | hTrx.v3-CT4-PSBD-His | 2.77 | 27.1 |
| | PSBD-TT | CT4 | pc0968 | hTrx.v3-CT4-PSBD-TT-His | 3.74 | 15 |
| | - | CT5 | pc0824 | hTrx.v3-CT5-His | 2.43 | 21.9 |
| | PSBD | CT5 | pc0886 | hTrx.v3-CT5-PSBD-His | 2.29 | 20.8 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| | PSBD-TT | CT5 | pc0969 | hTrx.v3-CT5-PSBD-TT-His | 3.56 | 31.7 |
| | PSBD | - | pc0908 | hTrx.v3-PSBD-His | 3.43 | 41.2 |
| | - | M12 | pc0811 | hTrx.v3-M12-His | 5.09 | 61.1 |
| | PSBD | M12 | pc0883 | hTrx.v3-M12-PSBD-His | 3.58 | 36.9 |
| | PSBD-TT | M12 | pc0965 | hTrx.v3-M12-PSBD-TT-His | 4 | 46.4 |
| | - | M21 | pc0812 | hTrx.v3-M21-His | 2 | 19 |
| | PSBD | M21 | pc0869 | hTrx.v3-M21-PSBD-His | 4.84 | 62.9 |
| | PSBD-TT | M21 | pc0966 | hTrx.v3-M21-PSBD-TT-His | 2.54 | 28.2 |
| | - | M30 | pc0792 | hTrx.v3-M30-His | 3.79 | 13.6 |
| | PSBD | M30 | pc0890 | hTrx.v3-M30-PSBD-His | 2.27 | 21.5 |
| | PSBD-TT | M30 | pc0973 | hTrx.v3-M30-PSBD-TT-His | 3 | 33.3 |
| | PSBD | M44 | pc0895 | hTrx.v3-M44-PSBD-His | 3.11 | 32.1 |
| | PSBD-TT | M44 | pc0967 | hTrx.v3-M44-PSBD-TT-His | 2.78 | 28.7 |
| | PSBD | Cpne1 | pc1304 | hTrx.v3-Cpne1-PSBD-His | 2.46 | 67.6 |
| | PSBD | Irgq | pc1307 | hTrx.v3-Irgq-PSBD-His | 4.01 | 109.6 |
| | PSBD | Aatf | pc1308 | hTrx.v3-Aatf-PSBD-His | 1.84 | 36.7 |
| After expressing by comprising MBP, cutting with TEV protease | CSTA | M30 | pc0690 | M30-GsGs-CystatinA-GG-His | 3.63 | 65.3 |
| | mCSTA | M30 | pc0733 | M30-mCSTA-His | 1.91 | 3.3 |
| | TrxA | M30 | pc0693 | M30-GsGs-TrxA-GG-His | 4.46 | 73.6 |
| | CSTA | M44 | pc0691 | M44-GsGs-CystatinA-GG-His | 6.73 | 50.4 |
| | TrxA | M44 | pc0692 | M44-GsGs-TrxA-GG-His | 3.22 | 22.5 |
| | PSBD | melanA | pc0988 | melanA-PSBD_G-His | 0.524 | 4.6 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
|  | CSTA | OVA27 | pc0688 | OVA-GsGs-CystatinA-GG-His | 3.1 | 4.7 |
|  | NDPK | OVA27 | pc0588 | OVA-Gs-NDPK | 9.52 | 19 |
|  | TrxA | OVA27 | pc0689 | OVA-GsGs-TrxA-GG-His | 3.1 | 4 |
|  | PSBD | survivin | pc0989 | survivin-PSBD_G-His | 0.591 | 3 |
| mCSTA | - | CT2 | pc0706 | mCSTA-CT2-His | 2.8 | 2.6 |
|  | - | CT3 | pc0707 | mCSTA-CT3-His | 4.63 | 30.1 |
|  | - | CT4 | pc0708 | mCSTA-CT4-His | 2.39 | 1.9 |
|  | - | CT5 | pc0709 | mCSTA-CT5-His | 2.86 | 19.4 |
|  | - | CT6 | pc0710 | mCSTA-CT6-His | 3.49 | 26.1 |
|  | - | M30 | pc0712 | mCSTA-M30-His | 3.35 | 20.1 |
|  | - | M30 | pc0723 | mCSTA-M30-His | 4.51 | 33.8 |
|  | - | M30 | pc0748 | mCSTA-GGGGG-M30-His | 2.7 | 21.6 |
|  | - | M44 | pc0720 | mCSTA-M44-His | 2.47 | 54.7 |
|  | - | OVA8.1 | pc0715 | mCSTA-OVA8.1-His | 10.99 | 35.2 |
|  | - | OVA8.1 | pc0747 | mCSTA-GGGGG-OVA8.1-His | 3.08 | 24.6 |
|  | - | OVA8.2 | pc0714 | mCSTA-OVA8.2-His | 2.04 | 15.3 |
|  | - | OVA8.3 | pc0713 | mCSTA-OVA8.3-His | 4.44 | 33.3 |
|  | - | OVA8.4 | pc0704 | mCSTA-OVA8.4-His | 3.78 | 20.8 |
| RPL7Am | PSBD | CT1 | pc0874 | RPL7Am-CT1-PSBD-His | 1.53 | 14.5 |
|  | PSBD | CT5 | pc0875 | RPL7Am-CT5-PSBD-His | 1.24 | 7.9 |
| Samp2a | PSBD | CT1 | pc0880 | Samp2a-CT1-PSBD-His | 0.29 | 2.2 |
|  | PSBD | CT5 | pc0881 | Samp2a-CT5-PSBD-His | 0.66 | 9.9 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| TE3.1 | - | CT2 | pc0804 | TE3.1-CT2-His | 2.91 | 11.1 |
| | - | CT24 | pc0807 | TE3.1-CT24-His | 2.31 | 24.5 |
| | - | CT25 | pc0808 | TE3.1-CT25-His | 1.81 | 36.1 |
| | - | CT4 | pc0805 | TE3.1-CT4-His | 3.64 | 40 |
| | - | M12 | pc0809 | TE3.1-M12-His | 3.9 | 52.7 |
| | - | M21 | pc0810 | TE3.1-M21-His | 3.6 | 46.7 |
| | - | M30 | pc0754 | TE3.1-M30-His | 2.87 | 13.3 |
| | - | M44 | pc0755 | TE3.1-M44-His | 2.94 | 25.6 |
| TE3.1-4 | - | M30 | pc0776 | TE3.1-4-M30-His | 3.69 | 9.2 |
| TM1112 | PSBD | CT1 | pc0878 | TM1112-CT1-PSBD-His | 1.33 | 15.3 |
| | PSBD | CT24 | pc0939 | TM1112-CT24-PSBD-His | 4.65 | 60.5 |
| | PSBD-TT | CT24 | pc0962 | TM1112-CT24-PSBD-TT-His | 3.02 | 42.3 |
| | PSBD | CT25 | pc0934 | TM1112-CT25-PSBD-His | 2.61 | 39.7 |
| | PSBD-TT | CT25 | pc0952 | TM1112-CT25-PSBD-TT-His | 3.07 | 12.9 |
| | PSBD | CT4 | pc0922 | TM1112-CT4-PSBD-His | 5.87 | 96.2 |
| | PSBD-TT | CT4 | pc0942 | TM1112-CT4-PSBD-TT-His | 4.43 | 62 |
| | PSBD | CT5 | pc0879 | TM1112-CT5-PSBD-His | 2.29 | 31 |
| | PSBD-TT | CT5 | pc0943 | TM1112-CT5-PSBD-TT-His | 3 | 42 |
| | PSBD | M12 | pc0903 | TM1112-M12-PSBD-His | 4.18 | 52.3 |
| | PSBD-TT | M12 | pc0961 | TM1112-M12-PSBD-TT-His | 4.54 | 56.7 |
| | PSBD | M21 | pc0933 | TM1112-M21-PSBD-His | 3.53 | 19.4 |
| | PSBD-TT | M21 | pc0951 | TM1112-M21-PSBD-TT-His | 5.33 | 75.2 |
| | PSBD | M30 | pc0923 | TM1112-M30-PSBD-His | 3.05 | 38.4 |
| | PSBD-TT | M30 | pc0940 | TM1112-M30-PSBD-TT-His | 2.76 | 33.9 |
| | PSBD | M44 | pc0924 | TM1112-M44-PSBD-His | 3.55 | 50.1 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| | PSBD-TT | M44 | pc0941 | TM1112-M44-PSBD-TT-His | 6.6 | 99.7 |
| TrxA | - | CT1 | pc0727 | TrxA-CT1-His | 2.44 | 15.6 |
| | - | CT2 | pc0728 | TrxA-CT2-His | 2.57 | 8.2 |
| | - | CT20 | pc0731 | TrxA-CT20-His | 2.57 | 19.5 |
| | - | CT24 | pc0737 | TrxA-CT24-His | 2.67 | 50.2 |
| | - | CT25 | pc0732 | TrxA-CT25-His | 3.48 | 19.5 |
| | - | CT4 | pc0729 | TrxA-CT4-His | 3.72 | 52.9 |
| | - | CT5 | pc0730 | TrxA-CT5-His | 1.85 | 26.6 |
| | - | M12 | pc0734 | TrxA-M12-His | 4.99 | 49.4 |
| | - | M21 | pc0736 | TrxA-M21-His | 3.3 | 40.6 |
| | - | M30 | pc0735 | TrxA-M30-His | 4.1 | 47.6 |
| | - | M44 | pc0738 | TrxA-M44-His | 2.37 | 76.6 |
| TrxA.v2 | - | CT2 | pc0816 | TrxA.v2-CT2-His | 1.03 | 10.7 |
| | - | CT20 | pc0791 | TrxA.v2-CT20-His | 2.93 | 35.1 |
| | - | CT5 | pc0821 | TrxA.v2-CT5-His | 0.85 | 6.4 |
| | - | M30 | pc0802 | TrxA.v2-M30-His | 4.05 | 20.3 |
| TT-hCSTA | - | CT24 | pc0785 | TT-Gs-hCSTA-CT24-His | 2.56 | 26.6 |
| | PSBD | CT24 | pc0931 | TT-Gs-hCSTA-CT24-PSBD-His | 4.08 | 50.6 |
| | PSBD | CT25 | pc0932 | TT-Gs-hCSTA-CT25-PSBD-His | 2.97 | 44.6 |
| | - | CT5 | pc0783 | TT-Gs-hCSTA-CT5-His | 1 | 6.8 |
| | PSBD | CT5 | pc0935 | TT-Gs-hCSTA-CT5-PSBD-His | 3.27 | 36.6 |
| | - | M12 | pc0787 | TT-Gs-hCSTA-M12-His | 4.35 | 52.2 |
| | PSBD | M12 | pc0926 | TT-Gs-hCSTA-M12-PSBD-His | 6.98 | 97 |
| | - | M21 | pc0788 | TT-Gs-hCSTA-M21-His | 2.7 | 4.5 |
| | PSBD | M21 | pc0927 | TT-Gs-hCSTA-M21-PSBD-His | 3.26 | 40.7 |
| | - | M44 | pc0746 | TT-Gs-hCSTA-M44-His | 2.49 | 19.9 |
| | PSBD | M44 | pc0929 | TT-Gs-hCSTA-M44-PSBD-His | 5.91 | 88.6 |

(continued)

| Result of preparing of neoantigen fusion proteins by each carrier protein | | | | | | |
|---|---|---|---|---|---|---|
| CP1 | CP2 | Neoantigen | Lot No | Construction | Prepared antigen concentration (mg/mL) | Prepared amount (mg) |
| TT-hTrx.v3 | PSBD | CT24 | pc0957 | TT-hTrx-CT24-PSBD-His | 4.72 | 59.2 |
| | PSBD | CT25 | pc0949 | TT-hTrx-CT25-PSBD-His | 2.7 | 23.7 |
| | PSBD | CT4 | pc0947 | TT-hTrx-CT4-PSBD-His | 4.98 | 66.2 |
| | PSBD | CT5 | pc0948 | TT-hTrx-CT5-PSBD-His | 3.27 | 39.2 |
| | PSBD | M12 | pc0953 | TT-hTrx-M12-PSBD-His | 7.32 | 107.6 |
| | PSBD | M21 | pc0954 | TT-hTrx-M21-PSBD-His | 2.33 | 27.6 |
| | PSBD | M30 | pc0955 | TT-hTrx-M30-PSBD-His | 3.6 | 49.3 |
| | PSBD | M44 | pc0956 | TT-hTrx-M44-PSBD-His | 4.4 | 64.2 |
| TT-TM1112 | PSBD | CT24 | pc0960 | TT-TM1112-CT24-PSBD-His | 3.12 | 45.9 |
| | PSBD | CT25 | pc0964 | TT-TM1112-CT25-PSBD-His | 2.4 | 35.8 |
| | PSBD | CT4 | pc0936 | TT-TM1112-CT4-PSBD-His | 3.45 | 44.9 |
| | PSBD | CT5 | pc0959 | TT-TM1112-CT5-PSBD-His | 3.59 | 52 |
| | PSBD | M12 | pc0950 | TT-TM1112-M12-PSBD-His | 4.56 | 61.6 |
| | PSBD | M21 | pc0963 | TT-TM1112-M21-PSBD-His | 2.04 | 25.7 |
| | PSBD | M30 | pc0937 | TT-TM1112-M30-PSBD-His | 3.99 | 54.2 |
| | PSBD | M44 | pc0958 | TT-TM1112-M44-PSBD-His | 4.02 | 56.3 |

## Example 3. Preparing of recombinant anticancer vaccines for treatment

### 3-1. Preparing of vectors

When a fusion protein is synthesized, if the insert length is short (within 400mer), 6 oligos with a length of 60-90 nucleotides were obtained and then overlap PCR was performed, and it the length is long, a sequence with restriction enzyme sites at the front and the rear was secured by gene synthesis. The primer and backbone sequences required for preparing each construct were summarized in Table 5 and Table 6. After treating restriction enzyme to the corresponding sequence and backbone vector for 1 hour, gel extraction was performed, and after treating ligase to the obtained gene fragment for 1 hour, *E. coli* transformation was performed.

The fusion protein sequence consists of carrier protein, epitopes, and a tag sequence for purification. All the sequences were prepared by overlap PCR or gene synthesis and then inserted into vectors. The epitope sequences might be continuously changed according to sequencing analysis of carcinomas, so after determining the sequences and order of the fusion proteins, it was designed that each epitope sequence is flanked by restriction enzyme sites, making it easy to construct the vector. In order to insert an additional sequence such as epitopes, and the like, two single base sequences (two oligo base sequences, some of which are complementary to each other) of 5' > 3' , 3' > 5 were attached through an annealing process, and then the vector was manufactured by using the annealed sequence as an insert. When the additional sequence became longer, they were prepared by increasing the number of the inserts.

For PCR, a mixture of primer, template, polymerase premix and DW was prepared, and a series of process were performed, starting from denaturation at 95°C for 1 minute, followed by 25 cycles of 3 steps: 95°C for 10 seconds, 60°C for 10 seconds and 72°C for 30 seconds, and a final extension at 72°C for 2 minutes, and 12°C for 5 minutes in a PCR machine. The corresponding sequences and the backbone vector were treated with restriction enzymes for 1 hour, gel extraction was performed, and the obtained gene fragments was treated with ligase for 1 hour, and then *E. coli* Transformation was conducted. When the insert was prepared by oligo annealing, two complementary oligo sequences were mixed at the same concentration of 1uM and it was set to 95°C 5 minutes, lowered to 50°C at ramp rate 0.1°C /sec, and then incubated for 10 minutes. The primers, backbone sequences and restriction enzyme sites required for preparing each construct were summarized in Table 5.

First, all the prepared vector backbones were derived from pMAL-c2x, pRK793, pET-Duet and pET28a(+)., and in case of pET28a(+), in order to change the restriction enzyme site, PCR was performed using the pET-Duet vector as a template and backbone F1 and backbone R1 primers, and then the pET-Backbone vector, which was primarily utilized as a backbone vector, and could be used as an enzyme site different from the conventional, was prepared by inserting a fragment cut using restriction enzyme XbaI and XhoI sites into pET-28a(+).

In addition, in order to express the epitope by Ubiquitin fusion, pUB-E1 vector was made: PCR was performed with UB_NdeI F and UB_SalI R using pTr-Ub-E1 fragment of which gene synthesis was performed in Cosmogenetech as a template, and then the PCR product and pET-Backbone vector made previously were cut with NdeI-SalI and ligated. This pUB-E1 vector was used for preparing other vectors using NdeI-SacI enzyme sites.

The preparation process of vectors for expressing the two fusion proteins (hTrx-epitope-PSBD, TM1112-epitope-PSBD) used in the evaluation of anticancer efficacy was as follows. First, in case of the hTrx fusion vector (All the hTrx sequences used in the evaluation of anticancer efficacy are designed as the form of hTrx.v3, which is the same as hTrx.v3 of Table 2), it was prepared by inserting various epitopes into a backbone vector which is designed to insert an epitope using two BsaI sites located between two carrier proteins in order of 'hTrx-BsaI enzyme site-PSBD-His' . For preparing of the vector, a few steps of overlap PCR were performed. The first overlap PCR was performed with two primers and two templates : the first template which was made by PCR using hTrx.v1 synthesized in Cosmogenetech as a template and two primers, hT NdeI F (GCGCATATGGTTAAACAAATTGAGTCGAAAAC: SEQ ID NO: 108) and hTrx.v3 R (TATCATTTTA GATGGACCAGACCAAGTGGCTGAGAAGTCAACTACGAC: SEQ ID NO: 109), the second template which was made by PCR using the hTrx.v1 sequence as a template and two primers, hTrx.v3 F (CACTTGGTCTGGTCCATCTAAAATGA TAAAGCCTTTTTTCCACTCGTTGAG: SEQ ID NO: 110) and hT BsaI R1 (TGAGACCCAGCGTTGCGGGTCTCTTCCTC CACCACCACCGACCAGTT: SEQ ID NO: 111), and two primers which are aforementioned hT NdeI F and BsaI R1. The second overlap PCR was performed using the first overlap PCR product as the first template, and the second template which was made by PCR using the PSBD sequence in which gene synthesis was performed in Cosmogenetech as a template and two primers, BsaI F (agagaccCGCAACGCTGggtctcaGGTGGTGGTGgaGgaGTTATCGCT: SEQ ID NO: 100) and PSBD R (CGACGAGCTCTCAATGGTGGTGATGGTGATGTCCGCCAGCCAGCCACGCGTC GATGTCTTCTTTCAGAACACGG: SEQ ID NO: 101) with two primers, hT NdeI F and PSBD. The second overlap PCR product and pET-backbone vector, which was made based on the pET-28 and pET-Duet, were cut with NdeI-SacI respectively and ligated, and thereby, the hTrx-BsaI enzyme site-PSBD-His vector was prepared.

Later, epitope expressing vectors prepared based on the hTrx-BsaI enzyme site-PSBD-His vector showed better physical properties in expression of various epitopes than other carrier proteins, but in MASS analysis, a phenomenon that the 27th glutamine of PSBD was cleaved was observed, so based on the paper that the corresponding phenomenon was reduced when the corresponding sequence was substituted with A/S/T/G, the stability of each substituent was confirmed through TEV cleavage after expression of MBP-TEV-PSBD, but single expression did not show a significant effect, so the N27G substituent expected to be most efficient was named PSBD_G and prepared. For preparing of the corresponding vector, the hTrx.v3-BsaI-PSBD_G-His vector was prepared by ligation by cutting with KpnI-BlpI with hTrx-BsaI enzyme site-PSBD-His which was the original vector to be linked after PCR using the original hTrx-BsaI enzyme site-PSBD-His vector as a template, and G (TCAGGGTACCGGTAAAGGTGGCCGTGTTCTGAAAGAAGACATCG: SEQ ID NO: 142) and T7 R 63 (TTCGCCAATCCGGATATAGTTCCTCCTTTC: SEQ ID NO: 120) as primers.

The vectors substituted with PSBD_G based on hTrx.v3-BsaI-PSBD_G-His showed a reduced effect, but in order to further supplement physical properties, a form of a GS linker of 10 amino acids in total that is extended from the linker length between the epitope and carrier protein, GGGGS, was prepared. Using the first template which was made by PCR using the hTrx.v3-BsaI-PSBD_G-His vector as a template and T7 F 63 (GTCCGGCGTAGAGGATCGAGATCTC:

SEQ ID NO: 131) and G10R (tgagaccCAGCGTTGCGggtctctgcCgcCaCCgCCaCCgcttcCACCACCACCGACCAGTT CATTAATCGTAGCT TCTAAT: SEQ ID NO: 143) as primers, and in the same manner, using the second template which was made by PCR using the hTrx.v3-BsaI-PSBD_G-His vector as a template and G10F (agagaccCGCAACGCTGggtct caGGcGGTGGcGgtGgtagcGGTGGTGgaGgaGTTATCGCTATGCCGTCCGTAC GCAAAT: SEQ ID NO: 144) and T7 R 63 (TTCGCCAATCCGGATATAGTTCCTCCTTTC: SEQ ID NO: 120) as primers, and using T7 F 63 and T7 R 63 as primers, overlap PCR was performed, and the overlap PCR product and the hTrx.v3-BsaI-PSBD_G-His vector were cut with BglII-SacI respectively and ligated, and thereby, the hTrx.v3-G10_BsaI-PSBD_G-His vector was prepared.

Then, in case of the TM1112 fusion vector, the first prepared platform was TM1112-BsaI-PSBD-His, which was prepared in the process of screening various carrier proteins located in the front of the epitope in order of carrier protein 1-epitope-carrier protein 2-his with a vector designed to allow inserting an epitope using two BsaI sites located between two carrier proteins. For preparing of the vectors, overlap PCR was performed using the first template, which was made by PCR with the base sequence of TM1112 synthesized in Cosmogenetech as template and TM F (TAGCGCATATGGAAGT TAAAATCGAGAAACCGACTCCGGAAAA: SEQ ID NO: 98) and TM 1R (TGAGACCCAGCGTTGCGGGTCTCTTCCTC CACCACCACCGAACAGGTT: SEQ ID NO: 99) as primers, and the second template, which was made by PCR using the PSBD sequence of which gene synthesis was performed in Cosmogenetech as a template and BsaI F and PSBD R as primers, with the corresponding TM F and PSBD R as primers. The overlap PCR product and pET-backbone vector were cut with NdeI-SacI respectively and ligated, and thereby, the TM1112-BsaI-PSBD-His vector was prepared.

In addition, the epitope expressing vectors prepared based on TM1112-BsaI-PSBD-His showed better physical properties in expression of various epitopes than other carrier proteins, but in the same manner as the hTrx vector, a phenomenon in which the 27th glutamine of PSBD was cleaved was observed, so based on the paper that the corresponding phenomenon was reduced when the corresponding sequence was substituted with A/S/T/G, the stability of each substituent was confirmed through TEV cleavage after expression of MBP-TEV-PSBD, but single expression did not show a significant effect, so the N27G substituent expected to be the most effective was named PSBD_G and prepared. For preparing of the corresponding vector, with TM1112-BsaI-PSBD-His as a vector, the TM1112-BsaI-PSBD_G-His vector was prepared by ligation by cutting with KpnI-BlpI with hTrx.v3-BsaI-PSBD_G-His in which PSBD was substituted with G previously as the insert.

The vectors substituted with PSBD_G based on TM1112-BsaI-PSBD_G-His showed an effect of reducing cleavage, but in order to further supplement physical properties, a form of a GS linker of 10 amino acids in total that is extended from the linker length between the epitope and carrier protein, GGGGS, was prepared. Using the first template, which was made by PCR using the TM1112-BsaI-PSBD_G-His vector as a template and T7 F 63 (GTCCGGCGTAGAG GATCGAGATCTC: SEQ ID NO: 131) and TM_G10_R (TGAGACCCAGCGTTGCGGGTCTCTTCCTCCACCAC CACCGCTTCCACCACCACCGAACAGGTTGTAGTGCATACGA ACAGG: SEQ ID NO: 145) as primers, and the second template, which was made by PCR using the pc1068 vector as a template and TM_G10_F (AGAGACCCG CAACGCTGGGTCTCAGGTGGCGGCGGTGGTAGCGGTGGTGGAGGAGTTATCGCTATGCCGTCCGTAC GCAAAT: SEQ ID NO: 146) and T7 R 63 (TTCGCCAATCCGGATATAGTTCCTCCTTTC: SEQ ID NO: 120) as primers, with T7 F 63 and T7 R 63 as primers, overlap PCR was performed. The overlap PCR product and the TM1112-BsaI-PSBD_G-His vector were cut with BglII-SacI respectively and ligated, and thereby, the TM1112-G10_BsaI-PSBD_G-His vector was prepared.

10 kinds of anticancer vaccines and other epitope expressing vectors used as purified examples were prepared by ligation of two oligos annealed on the BsaI site.

In case of TM-M30-PS, it was prepared by ligation of the linearied TM1112-BsaI-PSBD-His vector cut by BsaI and the annealing product of two nucleotide oligomers, M30 F (aGgaCCGAGCAAACCGTCGTTTCAGGAATTTGTG GACTGGGAAAACGTGTCGCCGGAACTGAaCAGCACCGATCAG CCGTTTCTG: SEQ ID NO: 102) and M30 R (CACC CAGAAACGGCTGATCGGTGCTGtTCAGTTCCGGCGACACGTTTTCCCAGTCCACAAATTCCTGAAACGACGG TTTGCTCGG: SEQ ID NO: 103).

In case of TM-CT5-PS, the insert was prepared through overlap PCR with two PCR products as templates: the first template was made by PCR with the TM sequence gene-synthesized in Cosmogenetech as a template and TM F and TM R5 (GCTCACCATAAACAGCTGCGGCATAATCGCCACGCTTTCCAGATAAATTCCTCCACCACCACCGAA CAGGTTGT: SEQ ID NO: 104) as primers, and the second template was made by PCR with the PSBD sequence gene-synthesized in Cosmogenetech as a template and CT5 F2 (GCGATTATGCCGCAGCTGTTTATGGTGAG CAAAGGCGGTGGTGGAGGAGTTATCGCTATGCCGTCCGTACGCAAAT ATGCACGTGAAA: SEQ ID NO: 105) and PSBD R as primers. Overlap PCR was performed using these two PCR products as templates and TM F and PSBD R as primers, and the overlap PCR product and pET-backbone vector were treated with the restriction enzyme of NdeI-SacI and ligated to prepare the vector.

The hT-M12-PS was prepared by cutting the hTrx-BsaI-PSBD-His vector with BsaI, annealing two nucleotide oligomers of M12 F (aGgaACCCCGCCGCCGGAAGAAGCGATGCCGTTTGAATTTAATGGTCCGGCGCAGGGTGAT CATAGCCAGCCGCCG CTGCAGGTG: SEQ ID NO: 115) and M12 R (CACCCACCTGCAGCGGCGGCTGGCTATGAT CACCCTGCGCCGGACCATTAAATTCAAACGGCATCGCTTCTTCCGG CGGCGGGGT: SEQ ID NO: 116), and joining the previously cut vector with ligase.

The hT-CT25-PS was prepared by cutting the hTrx-BsaI-PSBD-His vector with BsaI, and annealing two nucleotide oligomers of CT25 F (aGgaCTGCATCTGGAAGAAACCCTGGCGGGCTTTTGGGCGCGCCTGCTG GAAcgc: SEQ ID NO: 117) and CT25 R (CACCgcgTTCCAGCAGGCGCGCCCAAAAGCCCGCCAGGGTTTCTTCCA GATGCAG: SEQ ID NO: 118), and then joining the previously cut vector with ligase.

In case of the TT-hT-M30-PS, the first template, hT-M30-PS, is prepared by cutting the hTrx-BsaI-PSBD-His vector with BsaI, annealing two nucleotide oligomers of M30 F (aGgaCCGAGCAAACCGTCGTTTCAGGAATTTGTG GACTGGGAAAACGTGTCGCCGGAACTGAaCAGCACCGATCAG CCGTTTCTG: SEQ ID NO: 102) and M30 R (CACC CAGAAACGGCTGATCGGTGCTGtTCAGTTCCGGCGACACGTTTTCCCAGTCCACAAATTCCTGAAACGACGG TTTGCTCGG: SEQ ID NO: 103), and then joining the previously cutvector with ligase. In order to attach the front TT sequence, PCR was performed with the hT-M30-PS as a template, and htrx TT F (GCAAATTTATTGGCATTACC GAACTGGGCGGCGGCGGGTCTATGGTTAAACAAATTGAGTCGAAAACCG: SEQ ID NO: 119) and T7 R 63 as primers. And PCR was performed once again with the previous PCR product as a template, and TT F (GCGGCATATG CAGTATATTAAAGCGAACAGCAAATTTATTGGCATTACCGAACTG: SEQ ID NO: 121) and T7 R 63 primers, and then the vector was prepared through the NdeI-SacI site together cut and ligated with the pET-Backbone vector.

In case of TT-hT-CT5-PS, the first template, hT-CT5-PS, is prepared by cutting the previously prepared hT-BsaI-PS with BsaI, annealing two nucleotide oligomers of CT5 F (aGgaATTTATCTGGAAAGCGTGGCGATTATGCCG CAGCTGTTTATGGTGAGCAAA: SEQ ID NO: 122) and CT5 R (CACCTTTGCTCACCATAAACAGCTGCGGCA TAATCGCCACGCTTTCCAGATAAAT: SEQ ID NO: 123), and then joining the previously cut vectorwith ligase. Next, in order to attach the front TT sequence, PCR was performed with htrx TT F and T7 R primers. And then PCR was performed once again with TT F and T7 R primers, and then the vector was prepared through the NdeI-SacI site cut and ligated with the pET-Backbone vector.

In case of TT-CS-M21-PS, CSTA-OVA was prepared by cutting the PCR product, which was obtained by performing overlap PCR using the first fragment which was made by PCR using CSTA as a template and CSTA F (gc gaCTAGtAATAATTTTGTTTAACTTTAAGAAGGAGATATACCATGATCCCAGGAGGTCTGTCTGAAGCTAAGcc: SEQ ID NO: 124) and CS_OVA R (CTGTTCCAGACCGCTAACTTCATCGGATCCACCACCACCAAAACCCGTCAGCTCAT CATCTTT: SEQ ID NO: 125), the second fragment which was made by PCR using the OVA sequence as a template and CS_OVA F (GGTGGATCCGATGAAGTTAGCGGTCTGGAACAG: SEQ ID NO: 126) and OVA R (GACGAGCTCTCAGT GATGATGGTGGTGATGACCTCCACTAGACATAACATTGCTGCTGGTCCATTCGGT: SEQ ID NO: 127), and CSTA F and OVA R as primers and with SpeI-SacI and joining the PCR product and the pET-backbone with XbaI-SacI and ligase. After that, CSTA-M21 was prepared by cutting the previous CSTA-OVA and the DNA fragment which was made by PCR using the sequence made by annealing two oligomers, M21 F (AGGAAGCAGCCCGGATGAAGTGGCGCTGGTT GAAGGTGTGCAGAGCCTGGGTTTTACCTATCTGCGTCTGAAAGAT AATTATATG: SEQ ID NO: 147) and M21 R (C ACCCATATAATTATCTTTCAGACGCAGATAGGTAAAACCCAGGCTCTGCACACCTTCAACCAGCGCCACTTCATC CGGGCTGCT: SEQ ID NO: 148), as a template and M21 BamHI F (GCGGGATCCAGCAGCCCGGATGAAGTGGC: SEQ ID NO: 128) and M21 SacI R (CGCGAGCTCTCAGTGATGATGGTGGTGATGACCTCCCATATAATTATCTTTCAGACG CAGATAGGTAAAACCC: SEQ ID NO: 129) as primers, with the BamHI-SacI site and ligated. In addition, after performing PCR using the CSTA-M21 vector as a template and hCS TT F2 (GCGCATATGGGCGGCGGCGGGTCTATGATCCCAG GAGGTCTGTCTGAAG: SEQ ID NO: 149) and T7 R 63 primers, and then performing PCR once again using the PCR product as a template and TT F (GCGCATATGCAGTATATTAAAGCGAACAGCAAATTTATTGGCATTACC GAACTGGGCGGCGGCGGGTCTATGAT: SEQ ID NO: 121) and T7 R 63 primers, the PCR product and the pET-Backbone vector were cut with the NdeI-SacI site and ligated, the TT-CS-M21 vector was prepared. Furthermore, the TT-CS-M21-PS vector was prepared by cutting the overlap PCR product which was using the first fragment which was made by PCR using the TT-CS-M21 vector as a template and T7 F 63 and CS-M21 R primers (GCGTACGGACGGCATAGC GATAACTCCTCCACCACCACCCATATAATTATCTTTCAGACGCAGATAGGTA: SEQ ID NO: 132), the second fragment which was made by PCR using the PSBD sequence as a template and PSBD F2 (GGTGGTGGTGGAGGAGTTATCGC TAT: SEQ ID NO: 133) and T7 R 63 primers, with T7 F and T7 R as primers, and the pET-Backbone with NdeI-SacI, and ligated.

In case of TT-CS-CT5-PS, CSTA-CT5 was prepared by ligation of the CSTA-OVA fragment which cut by with BamHI-SacI and the other DNA product annealed by CT5 BamHI F (GATCCATTTATCTGGAAAGCGTGGCGAT TATGCCGCAGCTGTTTATGGTGAGCAAAGGCGGACATCACCATCACCA CCATTGAGAGCT: SEQ ID NO: 134) and CT5 SacI R (CTCAATGGTGGTGATGGTGATGTCCGCCTTTGCTCACCATAAACAGCTGCGGCATAATCGC CACGCTTTCCAGATA AATG: SEQ ID NO: 135). In addition, after performing PCR using the CSTA-CT5 vector as a template and hCS TT F and T7 R primers, and then performing PCR again using the PCR product as a template and TT F and T7 R primers, TT-CS-CT5 vector was prepared by cut and ligation of the final PCR product and the pET-Backbone vector with the NdeI-SacI site. In addition, the TT-CS-CT5-PS vector was prepared by cutting the overlap PCR product which was made using the first fragment which was made by PCR with the TT-CS-CT5 vector as a template and T7 F and CS-CT5 R primers, the second fragment which was made by PCR with the PSBD sequence as a template and PSBD F2 and T7 R primers, and the T7 F and T7 R as primers, and the pET-Backbone with NdeI-SacI, and ligated.

In case of TT-TM-M44-PS, the first vector was prepared by cutting TM1112-BsaI-PSBD-His with the BsaI and joining it with the DNA fragment made by annealing of two nucleotide oligomers of M44 F (aGgaGAATTTAAACATAT TAAAGCGTTTGATCGTACCTTTGCGAATAACCCGGGTCCGATGGTGGTGTTTGCGACC CCGGGTATG: SEQ ID NO: 137) and M44 R (CACCCATACCCGGGGTCGCAAACACCACCATCGGACCCGGGTTATTCGCAAAGGTACGAT CAAACGCTTTAATATG TTTAAATTC: SEQ ID NO: 138). Then in order to attach the front TT sequence, two PCR reactions were performed. The first PCR was done with the previously prepared vector as a template and htrx TT F and T7 R primers, and the second one was done using the first PCR product as a template and TT F and T7 R 63 primers. The last PCR product and pET-Backbone vector were cut and ligated with NdeI-SacI sites.

In case of TT-TM-CT25-PS, the first vector was prepared with the reaction, where TM1112-BsaI-PSBD-His vector was cut with BsaI and joining it with the DNA fragment made by annealing of two nucleotide oligomers of CT25 F (aGgaCTGCATCTGGAAGAAACCCTGGCGGGCTTTTGGGCGCGCCTGCTGGAAcgc: SEQ ID NO: 140) and CT25 R (CACCGcgTTCCAGCAGGCGCGCCCAAAAGCCCGCCAGGGTTTCTTCCAGATGCAG: SEQ ID NO: 141). Then in order to attach the front TT sequence, two PCR reactions were performed. The first PCR was done with the previously prepared vector as a template and htrx TT F and T7 R primers, and the second one was done using the first PCR product as a template and TT F and T7 R primers. The last PCR product and pET-Backbone vector were cut and ligated with NdeI-SacI sites.

The ten vectors used in evaluation of anticancer vaccine efficacy were prepared by ligation of the DNA fragment made by annealing two oligos and backbone vector cut by the BsaI site, like the vectors that are used in the embodiments for purification.

Among hTrx.v3-CT4-PSBD-His, hTrx.v3-CT5-PSBD-His, and hTrx.v3-CT24-PSBD-His, hTrx.v3-CT5-PSBD-His was the same as hT-CT5-PS, and hTrx.v3-CT4-PSBD-His and hTrx.v3-CT24-PSBD-His were prepared by cutting the previously prepared hT-BsaI-PS with BsaI, and joining the cut vector and two DNA fragments made by annealing two oligomer pairs, CT4 F (aGgaGGCGGCATTAGCGTGAAAGAACATATTGAAGTGAACGTGGTGCCGCTgACC: SEQ ID NO: 156) and CT4 R (CACCGGTcAGCGGCACCACGTTCACTTCAATATGTTCTTTCACGCTAATGCCGCC: SEQ ID NO: 157) as one pair, and CT24 F (aGgaCCGCTGGAAGCGATGTATATGCAGTGGCCGGTGATTGCGGTGAA CAACgGC: SEQ ID NO: 158) and CT24 R (CACCGCcGTTGTTCACCGCAATCACCGGCCACTGCATATACATCGCTTC CAGCGG: SEQ ID NO: 159) as the other pair.

hTrx.v3-Cpne1-PSBD-His, hTrx.v3-Irgq-PSBD-His, and hTrx.v3-Aatf-PSBD-His were prepared by cutting the hTrx.v3-G10_BsaI-PSBD_G-His vector with BsaI, and joining it with the each pair of annealed DNA oligomers : Aatf F (CGGCCATGTGCTGAGCAAACTGCTGAGCTTTATGGCGCCGATTGATCATACCACCATGAGCGATGATGCGCG CACC GAACTGTTT: SEQ ID NO: 150) and Aatf R (CaCCAAACAGTTCGGTGCGCGCATCATCGCTCATGGTGGTAT GATCAATCGGCGCCATAAAGCTCAGCAGTTTGCT CAGCACATG: SEQ ID NO: 151) as a pair, and Cpne F (CGGC GGCAGCAACGGCGATCCGAGCAGCCCGTATAGCCTGCATTATCTGAGCCCGACCGGCGTGAACGAATATCTG ACCGCGCTG: SEQ ID NO: 152) and Cpne R (CaCCCAGCGCGGTCAGATATTCGTTCACGCCGGTCGGGCTCAGA TAATGCAGGCTATACGGGCTGCTCGGATCGCC GTTGCTGCC: SEQ ID NO: 153) as another pair, and Irgq F (CGG CCCGGGCGATAGCCAGAACGCGGCGAAAGCGCGCGATGAAACCGCGGCGCTGCTGAACAGCGCGGTGCTGGG C GCGGCGCCG: SEQ ID NO: 154) and Irgq R (CaCCCGGCGCCGCGCCCAGCACCGCGCTGTTCAG CAGCGCCGCGGTTTCATCGCGCGCTTTCGCCGCGTTCTGGCT ATCGCCCGG: SEQ ID NO: 155) as other pair.

Among TM1112-M44-PSBD-His, TM1112-M30-PSBD-His, TM1112-M12-PSBD-His, and TM1112-M21-PSBD-His, TM1112-M30-PSBD-His was the same as TM-M30-PS, and the others were completed by cutting TM1112-BsaI-PSBD-His with BsaI, and joining it with each pairs of annealed DNA fragment : M44 F and M44 R as one pair, and M12 F (aGgaACCCCGCCGCCGGAAGAAGCGATGCCGTTTGAATTTAATGGTCCGGCGCAGGGTGATCATAGC CAGCCGCCG CTGCAGGTG: SEQ ID NO: 115) and M12 R (CACCCACCTGCAGCGGCGGCTGGCTATGAT CACCCTGCGCCGGACCATTAAATTCAAACGGCATCGCTTCTTCCGG CGGCGGGGT: SEQ ID NO: 116) as another pair, and M21 F (aGgaAGCAGCCCGGATGAAGTGGCGCTGGTTGAAGGTGTGCAGAGCCTGGGTTTTACC TATCTGCGTCTGAAAGAT AATTATATG: SEQ ID NO: 147) and M21 R (CACCCATATAATTATCTTTCAGACGCAGA TAGGTAAAACCCAGGCTCTGCACACCTTCAACCAGCGCCACTTCATC CGGGCTGCT: SEQ ID NO: 148) as other pair.

For the ease of purification of each construct, His tag was attached to the back, and in order to minimize the effect of addition of this tag sequence on physical properties of the protein, two glycine linkers were inserted, and also, a linker of GGGGG or GGGGS. or both of them was inserted between the fusion protein and epitope for the same purpose.

As Comp.cell for cloning, DH5alpha strain (Enzynomics) was used, and heat shock was applied for 1 minute and 30 seconds, and then it was stabilized in LB media for 1 hour, and then selection was performed by spreading in solid media containing an antibiotic marker. After growing 1-2 colonies in 2ml LB media for 6-8 hours at 37°C overnight (12-16h), mini-prep was performed to obtain a vector, and then a portion of each vector was used for sequencing analysis.

[Table 4]

| Linkers | | |
|---|---|---|
| SEQ ID NO: | Linker name | Amino acid sequence |
| - | GS | GS |
| 70 | GGGGS | GGGGS |
| - | LE | LE |
| 71 | SSGG | SSGG |
| - | GG | GG |
| 72 | GGGGG | GGGGG |
| 73 | G10 | GGGGSGGGGG |

[Table 5]

| Backbone sequence | | |
|---|---|---|
| SEQ ID NO: | Carrier protein | Nucleotide sequence |
| 74 | Mouse CSTA(Cystatin A) | ATGATCCCAGGTGGTCTGACCGAAGCGCGCCCGGCGACCGCGGAAGTGCAGGAAATTGCGGATC GCGTGAAAGCGCAGCTGGAAGAAGAAACCAACGAAAAATATGAAATTTTTAAAGCCGTTCAGTA CAAAACCCAGGTCGTAGCGGGTGTGAACTATTTTATTAAAATGGATGTGGGCGGCGGCTGCTTT ACCCATATTAAAGTGTTTAAAGATCTGAGCGGCAAAAACAACCTGGAACTGACCGGCTATCAGA CCAACAAAACCGAAGATGATGAACTGACCTATTTT |
| 75 | human CSTA(codon optimized) | ATGATCCCAGGAGGTCTGTCTGAAGCTAAGCCTGCAACGCCGGAAATTCAAGAAATTGTTGACA AAGTGAAGCCGCAGCTGGAAGAGAAAACCAACGAAACCTACGGTAAGCTGGAAGCCGTTCAGTA CAAAACCCAGGTCGTAGCGGGTACCAATTACTATATCAAAGTGCGTGCGGGTGACAACAAGTAT ATGCACCTGAAAGTTTTCAAAAGCCTCCCGGGACAGAATGAAGATCTGGTCTTGACTGGTTATC AAGTGGACAAGAACAAAGATGAtGAGCTGACGGGTTTT |
| 76 | hTrx.v1 | ATGGTTAAACAAATTGAGTCGAAAACCGCCTTCCAAGAAGCTCTTGATGCTGCTGGTGATAAGT TGGTCGTAGTTGACTTCTCAGCCACTTGGTGCGGTCCATGCAAAATGATAAAGCCTTTTTTTCCA CTCGTTGAGTGAGAAGTATTCAAACGTAATTTTTTTTAGAAGTGGATGTCGATGATTCACAAGAT GTTGCAAGTGAGAGCGAAGTCAAGTCTATGCCCACGTTTCAATTTTTTCAAAAAAGGTCAGAAGG TGGGCGAGTTTTCAGGTGCTAATAAAGAAAAATTAGAAGCTACGATTAATGAACTGGTC |
| 77 | hTrx.v2 | ATGGTTAAACAAATTGAGTCGAAAACCGCCTTCCAAGAAGCTCTTGATGCTGCTGGTGATAAGT TGGTCGTAGTTGACTTCTCAGCCACTTGGTGCGGTCCATGCAAAATGATAAAGCCTTTTTTTCCA CTCGTTGAGTGAGAAGTATTCAAACGTAATTTTTTTTAGAAGTGGATGTCGATGATTGCCAAGAT GTTGCAAGTGAGTGCGAAGTCAAGTCTATGCCCACGTTTCAATTTTTTCAAAAAAGGTCAGAAGG TGGGCGAGTTTTCAGGTGCTAATAAAGAAAAATTAGAAGCTACGATTAATGAACTGGTC |
| 78 | hTrx.v3 | ATGGTTAAACAAATTGAGTCGAAAACCGCCTTCCAAGAAGCTCTTGATGCTGCTGGTGATAAGT TGGTCGTAGTTGACTTCTCAGCCACTTGGTCTGGTCCATCTAAAATGATAAAGCCTTTTTTTCCA CTCGTTGAGTGAGAAGTATTCAAACGTAATTTTTTTTAGAAGTGGATGTCGATGATTCACAAGAT GTTGCAAGTGAGAGCGAAGTCAAGTCTATGCCCACGTTTCAATTTTTTCAAAAAAGGTCAGAAGG TGGGCGAGTTTTCAGGTGCTAATAAAGAAAAATTAGAAGCTACGATTAATGAACTGGTC |

(continued)

| | SEQ ID NO: | Carrier protein | Nucleotide sequence |
|---|---|---|---|
| | Backbone sequence | | |
| | 79 | mCSTA.midGGGGG | ATGATCCCAGGTGGTCTGACCGAAGCGCGCCCGGCGACCGCGGAAGTGCAGGAAATTGCGGATC GCGTGAAAGCGCAGCTGGAAGAAGAAACCAACGAAAAATATGAAATTTTTAAAGCCGTTCAGTA CAAAACCCAGGTCGGCGGCGGCGGGGGGGGTGAACTATTTTATTAAAATGGATGTGGGCGGCGGC TGCTTTACCCATATTAAAGTGTTTAAAGATCTGAGCGGCAAAAACAACCTGGAACTGACCGGCT ATCAGACCAACAAAACCGAAGATGATGAACTGACCTATTTT |
| | 80 | Samp2a | ATGAAGATGATCAAGGTTAAAGTAGTTGGTCGTAACATCGAGAAAGAGATCGAATGGCGCGAGG GTATGAAAGTGCGTGACATTCTGCGTGCAGTTGGTTTCAACACCGAGTCTGCTGTAGCAAAAGT GAACGGTAAAGTCGTGCTGGAAGACGACGAAGTAAAAGATGGTGACTTCGTGGAAGTTATTCCG GTTTGGACT |
| | 81 | TE1 (Tenascin 625-715) | CCACCGAAAGACCTGGTTGTTACCGAGGTGACTGAGGAAACCGTTAATCTGGCCTGGGACAACG AAATGCGTGTCACCGAGTATCTGGTTGTGTATACTCCGACCCATGAAGGTGGCCTGGAAATGCA GTTCCGTGTTCCGGGCGATCAGACCAGCACCATCATCCAGGAACTGGAGCCAGGCGTGGAGTAT TTCATCCGTGTTTTTCGCCATCCTGGAAAATAAAAAAATCTATCCCGGTTTCCGCGCGTGTAGCGA CCTATCTGCCAGCACCG |
| | 82 | TE2 (Tenascin 716-804) | GAAGGCCTGAAATTCAAAAAGCATCAAAGAGACCTCCGTTGAAGTAGAATGGGACCCGCTGGACA TCGCTTTTGAAACCTGGGAAATCATTTTCCGCAACATGAACAAAGAGGATGAAGGTGAAATCAC GAAAAGCCTGCGTCGTCCGGAAACCTCTTATCGTCAGACTGGTCTGGCTCCGGGCCAGGAATAT GAAATCTCTCTGCACATCGTGAAAAACAACACTCGCGGTCCTGGTCTGAAACGTGTCACTACTA CTCGTCTGGAC |
| | 83 | TE3.1-TE4 (Tenascin 805-990) | GCTCCGTCCCAGATCGAAGTGAAGGACGTAACGGATACCACCGCACTGATCACTTGGTTCAAAC CGCTGGCAGAAATTGATGGCATTGAACTGACCTATGGCATCAAAGACGTGCCGGGTGACCGTAC CACCATTGATCTGACGGAAGATGAGAACCAGTATTCCATCGGCAACCTGAAACCGGACACGGAA TACGAAGTGTCTCTGATCTCCCGTCGCGGCGACATGTCCTCTAACCCGGCTAAAGAAACTTTCA CTACCGGTCTGGATGCACCGCGCAACCTGCGTCGTGTATCTCAGACCGATAACTCTATTACCCT GGAATGGCGTAATGGTAAAGCTGCGATCGACTCCTACCGTATCAAATACGCGCCGATCTCCGGT GGTGATCATGCAGAAGTAGATGTACCTAAATCTCAGCAGGCTACGACGAAAACCACTCTGACTG GCCTGCGTCCGGGTACTGAGTATGGTATCGGTGTTTCTGCTGTTAAAGAAGATAAAGAATCCAA CCCAGCGACCATCAACGCGGCTACTGAACTGGATACGCCAAAAGAT |
| | 84 | TE3.1 (Tenascin 805-894) | GCTCCGTCCCAGATCGAAGTGAAGGACGTAACGGATACCACCGCACTGATCACTTGGTTCAAAC CGCTGGCAGAAATTGATGGCATTGAACTGACCTATGGCATCAAAGACGTGCCGGGTGACCGTAC CACCATTGATCTGACGGAAGATGAGAACCAGTATTCCATCGGCAACCTGAAACCGGACACGGAA TACGAAGTGTCTCTGATCTCCCGTCGCGGCGACATGTCCTCTAACCCGGCTAAAGAAACTTTCA CTACCGGTCTGGAT |

(continued)

| SEQ ID NO: | Carrier protein | Nucleotide sequence |
|---|---|---|
| | Backbone sequence | |
| 85 | TE3.2-TE4 (Tenas-cin 803-990) | CTGGATGCTCCGTCCCAGATCGAAGTGAAGGACGTAACGGATACCACCGCACTGATCACTTGGT TCAAACCGCTGGCAGAAATTGATGGCATTGAACTGACCTATGGCATCAAAGACGTGCCGGGTGA CCGTACCACCATTGATCTGACGGAAGATGAGAACCAGTATTCCATCGGCAACCTGAAACCGGAC ACGGAATACGAAGTGTCTCTGATCTCCCGTCGCGGCGACATGTCCTCTAACCCGGCTAAAGAAA CTTTCACTACCGGTCTGGATGCACCGCGCAACCTGCGTCGTGTATCTCAGACCGATAACTCTAT TACCCTGGAATGGCGTAATGGTAAAGCTGCGATCGACTCCTACCGTATCAAATACGCGCCGATC TCCGGTGGTGATCATGCAGAAGTAGATGTACCTAAATCTCAGCAGGCTACGACGAAAACCACTC TGACTGGCCTGCGTCCGGGTACTGAGTATGGTATCGGTGTTTCTGCTGTTAAAGAAGATAAAGA ATCCAACCCAGCGACCATCAACGCGGCTACTGAACTGGATACGCCAAAAGAT |
| 86 | TE3.2 (Tenascin 803-893) | CTGGATGCTCCGTCCCAGATCGAAGTGAAGGACGTAACGGATACCACCGCACTGATCACTTGGT TCAAACCGCTGGCAGAAATTGATGGCATTGAACTGACCTATGGCATCAAAGACGTGCCGGGTGA CCGTACCACCATTGATCTGACGGAAGATGAGAACCAGTATTCCATCGGCAACCTGAAACCGGAC ACGGAATACGAAGTGTCTCTGATCTCCCGTCGCGGCGACATGTCCTCTAACCCGGCTAAAGAAA CTTTCACTACCGGTCTG |
| 87 | TE4 (Tenascin 895-990) | GCACCGCGCAACCTGCGTCGTGTATCTCAGACCGATAACTCTATTACCCTGGAATGGCGTAATG GTAAAGCTGCGATCGACTCCTACCGTATCAAATACGCGCCGATCTCCGGTGGTGATCATGCAGA AGTAGATGTACCTAAATCTCAGCAGGCTACGACGAAAACCACTCTGACTGGCCTGCGTCCGGGT ACTGAGTATGGTATCGGTGTTTCTGCTGTTAAAGAAGATAAAGAATCCAACCCAGCGACCATCA ACGCGGCTACTGAACTGGATACGCCAAAAGAT |
| 88 | TM1112 | ATGGAAGTTAAAATCGAGAAACCGACTCCGGAAAAACTGAAGGAACTGTCTGTCGAAAAGTGGC CGATCTGGGAGAAAGAAGTGTCTGAGTTCGACTGGTACTACGATACCAACCGTACTGCATACAT CCTGGAGGGCAAGGTTGAAGTTACCACCGAAGATGGTAAGAAATACGTTATCGAAAAAGGCGAC CTGGTAACTTTTCCTAAAGGCCTGCGTTCTCGTTGGAAAGTTCTGGAACCTGTTCGTATGCACT ACAACCTGTTC |
| 89 | TrxA.v2 | ATGAGCGATAAAATTATTCACCTGACTGACGACAGTTTTGACACGGATGTACTCAAAGCGGACG GGGCGATCCTCGTCGATTTCTGGGCAGAGTGGTCTGGTCCGTCTAAAATGATCGCCCCGATTCT GGATGAAATCGCTGACGAATATCAGGGCAAACTGACCGTTGCAAAACTGAACATCGATCAAAAC CCTGGCACTGCGCCGAAATATGGCATCCGTGGTATCCCGACTCTGCTGCTGTTCAAAAACGGTG AAGTGGCGGCAACCAAAGTGGGTGCACTGTCTAAAGGTCAGTTGAAAGAGTTCCTCGACGCTAA CCTGGCC |
| 90 | TrxA | MSDKIIHLTDDSFDTDVLKADGAILVDFWAEWCGPCKMIAPILDEIADEYQGKLTVAKLNIDQN PGTAPKYGIRGIPTLLLFKNGEVAATKVGALSKGQLKEFLDANLA |
| 91 | TTrx(Thioredo xin from T. africanus) | ATGAAAATCGAATACTTTAAGAATGACAAAaGCTCTGTTaGCAAAGCTATGCTGCCGAAAATCC AAACTATTGCAAAAAAACTTTGATATTGATATTGAGGTAATTGACGTTATAGAAAATCCTTCTTA TCCAGCTCAAAAACTGGTTTTTTACAGTACCAACAGTAATCATTCTGGATAAAGAATTTGAAATC AAACGtTTTGCACGtAATTTTAGTATTAGTGAaGTAATcAATACAATTGAACGTTACCTGGAAA TAAGTAATAAAGAA |
| 92 | PSBD | VIAMPSVRKYAREKGVDIRLVQGTGKNGRVLKEDIDAWLA |

(continued)

| Backbone sequence | | |
|---|---|---|
| SEQ ID NO: | Carrier protein | Nucleotide sequence |
| 93 | PSBD_G | VIAMPSVRKYAREKGVDIRLVQGTGKGGRVLKEDIDAWLA |

[Table 6]

Primer sequences

| SEQ ID NO: | Primer name | Nucleotide sequence |
|---|---|---|
| 94 | backbone F1 | GCG TCTAGAAATAATTTTGTTTAACTTTAAGAAGGAGATATACATATGGCAGATCTCAATTGGAT |
| 95 | backbone R1 | CAGCGGTTTCTTTACCAGACTCGAG |
| 96 | UB_NdeI F | GCGCATATGCAGATTTTTGTGAAAACCCTGACC |
| 97 | UB_SalI R | GACTAGTTCTAGAGTCGACGAGCTCTC |
| 98 | TM F | TAGCGCATATGGAAGTTAAAATCGAGAAACCGACTCCGGAAAA |
| 99 | TM 1R | AGAGACCCGCAACGCTGGGTCTCAGAACACAGGTTGTAGTGCATACGAACA |
| 100 | Bsal F | AGAGACCCGCAACGCTGGGTCTCAGGTGGTGGTGGAGGAGTTATCGCT |
| 101 | PSBD R | CGACGAGCTCTCAATGGTGGTGATGGTGATGTCCGCCAGCCAGCCACGCGTCGATGTCTCTTTCAGAACACGG |
| 102 | M30 F | AGGACCGAGCAAACCGTCGTTTCAGGAATTTGTGGACTGGGAAAAGCGTGTCGCCGGAACTGAACAGCACCGGATCAGCCGTTTCTG |
| 103 | M30 R | CACCCAGAAACGGCTGATCGGTGCGTGTTCAGTTCCGGCGACACGTTTTCCCAGTCCACAAATTCCTGAAACGACGGTTTGCTCGG |
| 104 | TM R5 | GCTCACCATAAACAGCTGCGGCATAATCGGCCACGCTTTCCAGATAAATTCCTCCACCACCACCGAACAGGTTGT |
| 105 | CT5 F2 | GCGATTATGCCGCAGCTGTTTATGGTGAGCAAAGGCGGTGGTGAGGAGTTATCGCTATGCCGTCCGTACGCAAATATGCACGTGAAA |
| 106 | CT5 R2 | TTTACCGGTACCCTGAACCAGGCGGGGATATCAACGCCTTTTCACGTGCATATTGCGTACGGA |
| 107 | PSBD F | GCAAATATGCACGTGAAAAAGGCGTTGATATCCGCCCTGGTTCAGGGTACCGGTAAAAACGGCCGTGTTCTGAAAGAAGACATCGACGC |
| 108 | hT NdeI F | GCGCATATGGTTAAACAAATTGAGTCGAAAAC |
| 109 | hTrx.v3 R | TATCATTTTAGATGGACCAGACCAAGTGGCTGAGAAGTCAACTACGAC |
| 110 | hTrx.v3 F | CACTTGGTCTGGTCCATCTAAAATGATAAAGCCTTTTTCCACTCGTTGAG |
| 111 | hT Bsal R1 | TGAGACCCCAGCGTTGCGGGTCTCTTCCTCCACCACCACCGACCAGTT |

(continued)

| Primer sequences | | |
|---|---|---|
| SEQ ID NO: | Primer name | Nucleotide sequence |
| 112 | hT BsaI F | AGAGACCCGCAACGCTGGGTCTCAGGTGGTGGTGGAGGAGTTATCGCT |
| 113 | hT BsaI R2 | CCGGTACCCTGAACCAGGCGGATATCAACGCCTTTTTCACGTGCATATTTGCGTACGGACGGCATAGCGATAACTCCTCCACCACCACC |
| 114 | hT PSBD F | GCAAATATGCACGTGAAAAAGGCGTTGATATCCGCCTGGTTCAGGGTACCGGTAAAAACGGCCGTGTTCTGAAAGAAGACATCGACGC |
| 115 | M12 F | AGGAACCCCGCCGCCGGAAGAAGCGATGCCGTTTGAATTTAATGGTCCGGCGCAGGGTGATCATAGCCAGCCGCCGCTGCAGGTG |
| 116 | M12 R | CACCCACCTGCAGCGGCGGCTGGCTATGATCACCCTGCGCCGGACCATTAAATTCAAACGGCATCGCTTCTTCCGGCGGCGGGGT |
| 117 | CT25 F | AGGACTGCATCTGGAAGAAACCCTGGCGGGCTTTTGGGCGCGCCTGCTGGAACGC |
| 118 | CT25 R | CACCGCGTTCCAGCAGGCGCGCCCAAAAGCCCGCCAGGGTTTCTTCCAGATGCAG |
| 119 | htrx TT F | GCAAATTTATTGGCATTACCGAACTGGGCGGCGGCGGGTCTATGGTTAAACAAATTGAGTCGAAAACCG |
| 120 | T7 R | TTCGCCAATCCGGATATAGTTCCTCCTTTC |
| 121 | TT F | GCGGCATATGCAGTATATTAAAGCGAACAGCAAATTTATTGGCATTACCGAACTG |
| 122 | CT5 F | aGgaATTTATCTGGAAAGCGTGGCGATTATGCCGCAGCTGTTTATGGTGAGCAAA |
| 123 | CT5 R | CACCTTTGCTCACCATAAACAGCTGCGGCATAATCGCCACGCTTTCCAGATAAAT |
| 124 | CSTA F | GCGACTAGTAATAATTTTGTTTAACTTTAAGAAGGAGATATACCATGATCCCAGGAGGTCTGTCTGAAGCTAAGcc |
| 125 | CS_OVA R | CTGTTCCAGACCGCTAACTTCATCggatCCACCACCACCAAAACCCGTCAGCTCATCATCTTT |
| 126 | CS_OVA F | GGTGGat ccGATGAAGTTAGCGGTCTGGAACAG |
| 127 | OVA R | GACGAGCTCTCAGTGATGATGGTGGTGATGACCTCCACTAGACATAACATTGCTGCTGGTCCATTCGGT |
| 128 | M21 BamHI F | GCGGGATCCAGCAGCCCGGATGAAGTGGC |

(continued)

| Primer sequences | | |
|---|---|---|
| SEQ ID NO: | Primer name | Nucleotide sequence |
| 129 | M21 SacI R | CGC*GAGCTC*TCAGTGATGATGGTGGTGATGACCTCCCATATAATTATCTTTCAGACGCAGATAGGTAAAACCC |
| 130 | hCS TT F | GCGCATATGGGCGGCGGCGGGTCTATGATCCCAGGAGGTCTGTCTGAAG |
| 131 | T7 F | GTCCGGCGTAGAGGATCGAGATCTC |
| 132 | CS-M21 R | GCGTACGGACGGCATAGCGATAACTCCTCCACCACCACCCATATAATTATCTTTCAGACGCAGATAGGTA |
| 133 | PSBD F2 | GGTGGTGGTGGAGGAGTTATCGCTAT |
| 134 | CT5 BamHI F | GATCCATTTATCTGGAAAGCGTGGCGATTATGCCGCAGCTGTTTATGGTGAGCAAAGGCGGACATCACCATCACCACCATTGAGAGCT |
| 135 | CT5 SacI R | CTCAATGGTGGTGATGGTGATGTCCGCCTTTGCTCACCATAAACAGCTGCGGCATAATCGCCACGCTTTCCAGATAAATG |
| 136 | CS-CT5 R | GCGTACGGACGGCATAGCGATAACTCCTCCACCACCACCTTTGCTCACCATAAACAGCTGCGG |
| 137 | M44 F | AGGAGAATTTAAACATATTAAAGCGTTTGATCGTACCTTTGCGAATAACCCGGGTCCGATGGTGGTGTTTGCGACCCCGGGTATG |
| 138 | M44 R | CACCCATACCCGGGGTCGCAAACACCACCATCGGACCCGGGTTATTCGCAAAGGTACGATCAAACGCTTTAATATGTTTAAATTC |
| 139 | htrx TT F | GCAAATTTATTGGCATTACCGAACTGGGCGGCGGCGGGTCTATGGAAGTTAAAATCGAGAAACCGACTCC |
| 140 | CT25 F | AGGACTGCATCTGGAAGAAACCCTGGCGGGGCTTTTGGGCGCGCCTGCTGGAACGC |
| 141 | CT25 R | CACCGCGTTCCAGCAGGCGCGCCCAAAAGCCCGCCAGGGTTTCTTCCAGATGCAG |
| 142 | G | TCAGGGTACCGGTAAAGGTGGCCGTGTTCTGAAAGAAGACATCG |
| 143 | G10R | tgagaccCAGCGTTGCGggtctctgcCgcCaCCgCCaCCgcttcCACCACCACCGACCAGTTCATTAATCGTAGCTTCTAAT |

(continued)

| Primer sequences | | |
|---|---|---|
| SEQ ID NO: | Primer name | Nucleotide sequence |
| 144 | G10F | agagaccCGCAACGCTGggtctcaGGcGGTGGcGgtGgtagcGGTGGTGgaGgaGTTATCGCTAT GCCGTCCGTACGCAAAT |
| 145 | TM_G10_R | TGAGACCCAGCGTTGCGGGTCTCTTCCTCCACCACCACCGCTTCCACCACCACCGAACAGGTTGT AGTGCATACGAACAGG |
| 146 | TM_G10_F | AGAGACCCGCAACGCTGGGTCTCAGGTGGCGGCGGTGGTAGCGGTGGTGGAGGAGTTATCGCTAT GCCGTCCGTACGCAAAT |
| 147 | M21 F | aGgaAGCAGCCCGGATGAAGTGGCGCTGGTTGAAGGTGTGCAGAGCCTGGGTTTTACCTATCTGC GTCTGAAAGATAATTATATG |
| 148 | M21 R | CACCCATATAATTATCTTTCAGACGCAGATAGGTAAAACCCAGGCTCTGCACACCTTCAACCAGC GCCACTTCATCCGGGCTGCT |
| 149 | hCS TT F2 | GCGCATATGCAGTATATTAAAGCGAACAGCAAATTTATTGGCATTACCGAACTGGGCGGCGGCGG GTCTATGAT |
| 150 | Aatf F | CGGCCATGTGCTGAGCAAACTGCTGAGCTTTATGGCGCCGATTGATCATACCACCATGAGCGATG ATGCGCGCACCGAACTGTTT |
| 151 | Aatf RF | CaCCAAACAGTTCGGTGCGCGCATCATCGCTCATGGTGGTATGATCAATCGGCGCCATAAAGCTC AGCAGTTTGCTCAGCACATG |
| 152 | Cpne F | CGGCGGCAGCAACGGCGATCCGAGCAGCCCGTATAGCCTGCATTATCTGAGCCCGACCGGCGTGA ACGAATATCTGACCGCGCTG |
| 153 | Cpne R | CaCCCAGCGCGGTCAGATATTCGTTCACGCCGGTCGGGCTCAGATAATGCAGGCTATACGGGCTG CTCGGATCGCCGTTGCTGCC |
| 154 | Irgq F | CGGCCCGGGCGATAGCCAGAACGCGGCGAAAGCGCGCGATGAAACCGCGGCGCTGCTGAACAGCG CGGTGCTGGGCGCGGCGCCG |

EP 4 563 591 A1

(continued)

| Primer sequences | | |
|---|---|---|
| SEQ ID NO: | Primer name | Nucleotide sequence |
| 155 | Irgq R | CaCCCGGCGCCGCGCCCAGCACCGCGCTGTTCAGCAGCGCCGCGGTTTCATCGCGCGCTTTCGCC GCGTTCTGGCTATCGCCCGG |
| 156 | CT4 F | aGgaGGCGGCATTAGCGTGAAAGAACATATTGAAGTGAACGTGGTGCCGCTgACC |
| 157 | CT4 R | CACCGGTcAGCGGCACCACGTTCACTTCAATATGTTCTTTCACGCTAATGCCGCC |
| 158 | CT24 F | aGgaCCGCTGGAAGCGATGTATATGCAGTGGCCGGTGATTGCGGTGAACAACgGC |
| 159 | CT24 R | CACCGCcGTTGTTCACCGCAATCACCGGCCACTGCATATACATCGCTTCCAGCGG |

### 3-2. Culture

For the vector of which sequence was confirmed, transformation was performed in the clearcoli BL21(DE3) strain for culture. As electroporation was used for the comp.cell of the corresponding strain, the mixture of each DNA and competent cell were placed in a separate cuvette, and electric shock was applied. And stabilization was performed in LB media for 1 hour, and then selection was performed by spreading in solid media containing an antibiotic marker. After inoculating one colony in 3ml LB media containing antibiotics at 37°C overnight (16-20h), secondary inoculation was performed in 1.2L media the next morning. When OD was grown at a level of 0.7-1.2, IPTG solution was added for induction of 0.2mM concentration, and the culture temperature was lowered from 37°C > 25°C. After culturing overnight, the cells were recovered the next day.

### 3-3. Microbial cell recovery and lysing

The culture solution containing a recombinant anticancer vaccine expressed for treatment was transferred in a 1L bottle for centrifugation. The centrifugation condition was at 7000 rpm, 5±3°C, for 5 minutes. The supernatant was discarded and the precipitated microbial cells were recovered. After suspending the recovered microbial cells after centrifugation with a lysing solution (20 mM Sodium phosphate, 0.3M NaCl, 10 mM Imidazole, 1mM PMSF pH8.0), the cells were lysed using an ultrasonicator. After lysing, the cell lysate was transferred in a 50 mL bottle for centrifugation. The centrifugation condition was at 40,000xg, 5±3°C for 30 minutes, and the supernatant was purified by Akta pure system.

### 3-4. Purification

Purification of the recombinant anticancer vaccine for treatment was conducted using filtration and 2-step column chromatography.

Step 1: Metal ion affinity chromatography (IMAC)

Using 20 mM sodium phosphate / 0.3M sodium chloride / 10 mM imidazole buffer, a metal ion affinity column (Cobalt immobilized column) was equilibrated. After lysing, the centrifugation supernatant was filtered through a 0.22$\mu$m filter, and then loaded on the column, and washed with 20 mM sodium phosphate / 0.3M sodium chloride / 10 mM imidazole buffer (pH8.0). Subsequently, using 20 mM sodium phosphate / 0.3M sodium chloride / 150 mM imidazole buffer (pH8.0), the recombinant anticancer vaccine was eluted.

Step 2: Size exclusion chromatography (SEC)

The size exclusion chromatography column (Hiload Superdex G75) was equilibrated using phosphate buffer physiological saline solution. The elution eluted in the metal ion affinity column was loaded on the column, and then the ultraviolet absorbance (280 nm) was monitored, and fractions at an approximately 20kD elution point were collected.

Step 3: Sterilized filtration

In process control (anticancer vaccine protein concentration) for the obtained fractions was conducted. Whether additional concentration was required to satisfy the protein concentration target was determined. If needed, they were concentrated so that the final concentration of the protein was 2.0mg/mL or more.

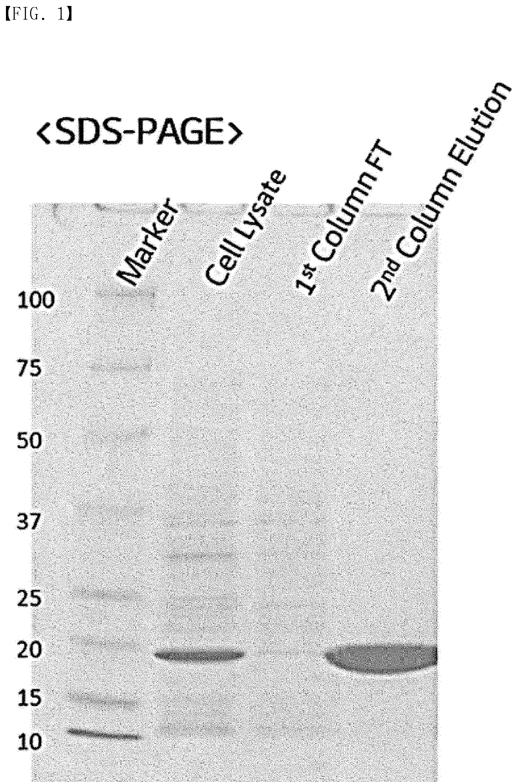

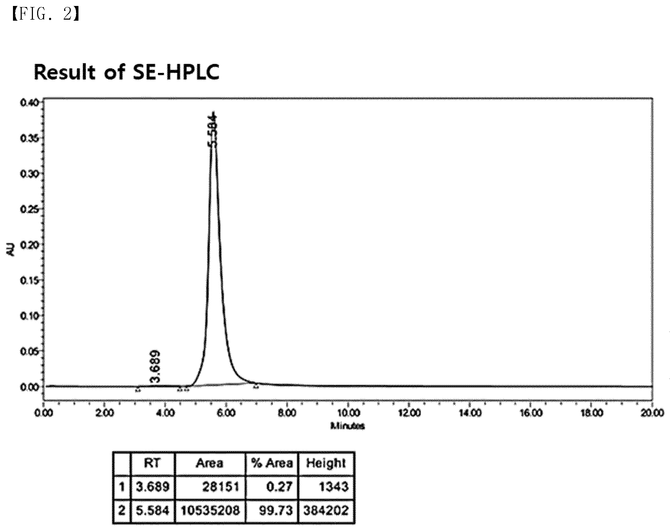

Finally, the anticancer vaccine protein was filtered (0.22,um), and distributed in a sterilized 1.5 mL polyethylene (Cyrotube) container. In this step, as in process control (IPC), a confirmation test (SDS-PAGE), a protein content (UV method), an endotoxin content (kinetic method), a purity test (SE-HPLC) were conducted, and the results were shown in FIG. 1, FIG. 2 and Table 7 (FIG. 1 is the result of the hT-CT25-PSBD, and FIG. 2 is the result of the TM-CT5-PSBD). The final sterilized anticancer vaccine active materials were stored at -70±10°C.

[Table 7]

| Test item | Protein concentration (mg/mL) | Produced amount (mg) | Purity test (%) | Endotoxin (EU/0.2mg) | Confirmation test | |
|---|---|---|---|---|---|---|
| Material name (lot No) | Abs. at 280nm | Abs. at 280nm | SE-HPLC | Kinetic | MS analysis | SDS-PAGE |

(continued)

| Test item | Protein concentration (mg/mL) | Produced amount (mg) | Purity test (%) | Endotoxin (EU/0.2mg) | Confirmation test | |
|---|---|---|---|---|---|---|
| TM-M12-PSBD(pc0903) | 4.33 | 59.3 | 100.0 | 2.74 | Confirmed | Confirmed |
| TM-M21-PSBD(pc0933) | 4.64 | 115.9 | 100.0 | 0.17 | Confirmed | Confirmed |
| TM-M30-PSBD(pc0923) | 3.06 | 15.3 | 100.0 | 0.62 | Confirmed | Confirmed |
| hT-M12-PSBD(pc0883) | 3.58 | 36.9 | 99.3 | 0.40 | Confirmed | Confirmed |
| hT-CT4-PSBD(pc0885) | 4.42 | 131.0 | 100.0 | 0.25 | Confirmed | Confirmed |
| hT-CT5-PSBD(pc0886) | 3.27 | 81.9 | 100.0 | 0.23 | Confirmed | Confirmed |
| hT-CT24-PSBD(pc0888) | 4.54 | 136.3 | 100.0 | 0.06 | Confirmed | Confirmed |
| hT-Cpne1-PSBD(pc1304) | 2.46 | 67.6 | 100.0 | 0.11 | N/A | Confirmed |
| hT-Iraq-PSBD(pc1307) | 4.01 | 109.6 | 100.0 | 0.05 | N/A | Confirmed |
| hT-Aatf-PSBD(pc1308) | 1.84 | 36.7 | 100.0 | 0.05 | N/A | Confirmed |
| TT-hT-M30-PSBD(pc0955) | 3.60 | 49.3 | 99.6 | 0.05 | Confirmed | Confirmed |
| TT-hT-CT5-PSBD(pc0948) | 3.27 | 39.2 | 99.6 | 2.07 | Confirmed | Confirmed |
| TT-CS-M21-PSBD(pc0927) | 3.26 | 40.7 | 100.0 | 0.06 | Confirmed | Confirmed |
| TT-CS-CT5-PSBD(pc0935) | 3.27 | 36.6 | 100.0 | 1.34 | Confirmed | Confirmed |
| TT-TM-M44-PSBD(pc0958) | 4.02 | 56.3 | 99.7 | 2.00 | Confirmed | Confirmed |
| TT-TM-CT25-PSBD(pc0964) | 2.40 | 35.8 | 99.8 | 2.79 | Confirmed | Confirmed |
| (In the above table, TM represents TM1112, and hT represents human Trx, and TT-ht represents Tetanus Toxoid - human Trx, and TT-CS represents Tetanus Toxoid - CSTA, and TT-TM represents Tetanus Toxoid- TM1112) | | | | | | |

**3-5. Formulation of recombinant anticancer vaccine proteins for treatment**

Formulation of recombinant anticancer vaccine proteins for treatment was conducted all in a biosafety cabinet (BSC). Based on the batch volume and anticancer vaccine protein concentration, the required volume of each anticancer vaccine active material was calculated. In addition, by adding buffer containing sodium chloride, phosphate (pH 7.4), and polysorbate 80, the combined anticancer vaccine proteins were diluted to the target concentration. After mixing sufficiently, the formulated anticancer vaccine for treatment was mixed with Poly IC and administered into an animal.

Table 8 below shows the final composition of the recombinant anticancer vaccine for treatment in an adjuvanted form.

[Table 8]

| Composition of adjuvanted recombinant anticancer vaccine formulation | | |
|---|---|---|
| Component | | Formulation for administration (1 mL /vial) |
| Active ingredient | Recombinant protein comprising a neoantigen | Based on a protein content : 0.2 ~ 1.0 mg 1 ~ 5 kinds of neoantigens : 0.2 mg each |
| Adjuvant | Poly IC | 0.5 mg |
| Excipient | Polysorbate 80 | 0.02 % |
| | Phosphate | 10 mM |
| | Sodium chloride | 150 mM |
| | Water for injection | Q.S* |
| *Sufficient amount to reach 1.0 mL | | |

**Example 4. Removal of endotoxin using size exclusion chromatography**

The host cell used in the present invention, *E.coli* is a gram-negative bacterium, and is a material which secrets endotoxin can cause a problem in safety, when injected in a body. Therefore, protein drugs using *E.coli* as a host cell are subject to thorough quality control for endotoxin, and an endotoxin removal process should be verified. Accordingly, in the present invention, using a size exclusion chromatography process, quality control was conducted with a trace amount of endotoxin.

The endotoxin of gram-negative bacteria is in a lipopolysaccharide (LPS) form, and has hydrophilic and hydrophobic properties, and forms micelles at a certain concentration and condition, and through formation of endotoxin micelles, the size of the endotoxin was increased. In the present invention, it was designed with a material with a molecular weight as small as possible so as to separate the endotoxin micelles and target protein in the size exclusion chromatography process, and as a result of actually collecting fractions according to retention time and analyzing the endotoxin content, the endotoxin content was lower from the fraction with a short retention time (large size) to the fraction with a long retention time (small size). Through the present process, it was confirmed that the fraction corresponding to the size of the anticancer vaccine protein contained a trace amount of endotoxin.

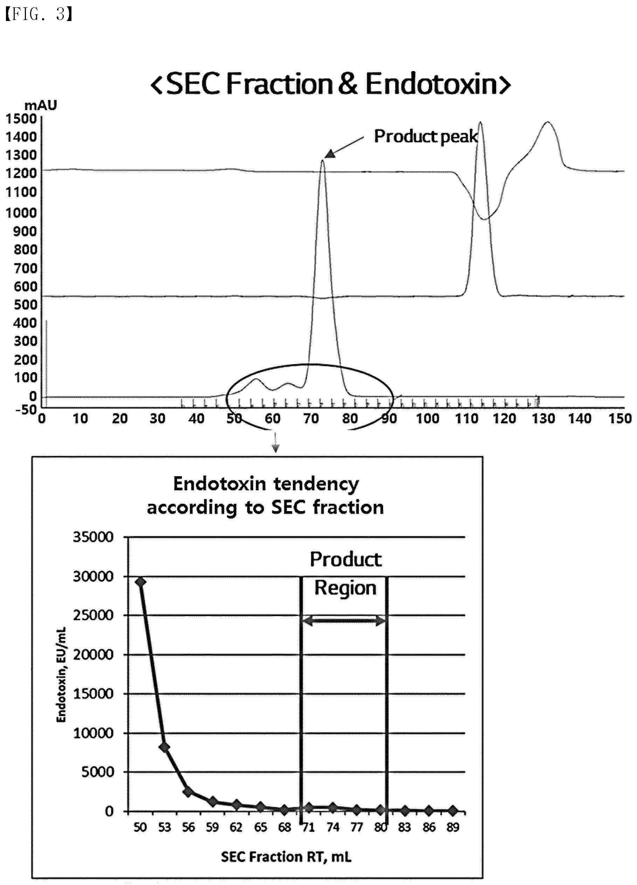

The endotoxin content according to the size exclusion chromatography fraction was shown in FIG. 3 and Table 9 (FIG. 3 is the result of the TrxA-M30-His, and is the arrangement to see the difference in endotoxin according to SEC fraction, and corresponds to the Lot No: pc0735 arrangement described in Table 3).

[Table 9]

| Unit process | | Endotoxin content (EU/mL) | Volume (mL) | Endotoxin total amount (EU) |
|---|---|---|---|---|
| Cell Extract | | 500,000 or more | 80 | 40,000,000 |
| 2nd SEC | Fraction #2 | 29346 | 3 | 88037 |
| | Fraction #3 | 8191 | 3 | 24573 |
| | Fraction #4 | 2505 | 3 | 7515 |
| | Fraction #5 | 1228 | 3 | 3684 |
| | Fraction #6 | 811 | 3 | 2434 |
| | Fraction #7 | 514 | 3 | 1542 |
| | Fraction #8 | 215 | 3 | 644 |
| | Fraction #9 | 465 | 3 | 1394 |
| | Fraction #10 | 474 | 3 | 1422 |
| | Fraction #11 | 201 | 3 | 603 |
| | Fraction #12 | 166 | 3 | 497 |
| | Fraction #13 | 90 | 3 | 271 |
| | Fraction #14 | 56 | 3 | 167 |

(continued)

| Unit process | | Endotoxin content (EU/mL) | Volume (mL) | Endotoxin total amount (EU) |
|---|---|---|---|---|
| | Fraction #15 | 37 | 3 | 111 |

## Example 5. Evaluation of immunogenicity of anticancer vaccines

In order to evaluate whether the anticancer vaccine composition prepared in the above example induced an immune response in a mouse, research was performed. This immune response was confirmed by ELISPOT of the interferon gamma (IFN-ɣ) concentration.

In addition, 1 at minimum to 4 at maximum of the anticancer vaccine proteins were mixed by 1-10 nmol each, and subcutaneous injection was performed. poly IC (invivogen) was mixed into the anticancer vaccine protein by 50 ug per mouse and administered, and administration was performed twice a week for 2 weeks. Immunogenicity was confirmed from the spleen of the mouse on 4~5 days after the last inoculation. This was described in detail as follows.

### 5-1. Mouse homogeneous anticancer model

6-week-old female C57BL/6 mice and Balb/c mice were purchased from Koatech and acclimatized for one week before starting the research. 100ul of the mouse melanoma cell line B16-F10 ($5X10^4$) or mouse colorectal cancer cell line CT26($1X10^6$) was subcutaneously transplanted into the right flank of the mouse. Before inoculation, cells were subcultured in DMEM or RPMI media in which 10% fetal bovine serum (FBS), 2mM L-glutamine were added 5 times or less. The cells were cultured in a 37°C, 5% CO2 incubator. When 80-90% cells were filled, the cells were harvested, and resuspended with a 1:1 mixture of serum-free media and Matrigel.

For the mouse, after cell transplantation, measurement was performed 3 times every week. For tumor measurement, the length and width of each tumor were measured using calipers, and the volume was calculated according to the formula: tumor volume ($mm^3$) = (width $(mm)^2$ X length (mm)/2). All tumors were measured between 5 and 10 days after inoculation, and group separation (8~10 mice/group) was performed. The average tumor volume of all animals was 50~100$mm^3$. The anticancer vaccine material (10nmol) and polyIC (50ug) were administered by 300ul by a subcutaneous injection twice a week for 2 weeks, and when the tumor volume reached 1000 ~ 2000$mm^3$, the tumor was continuously measured.

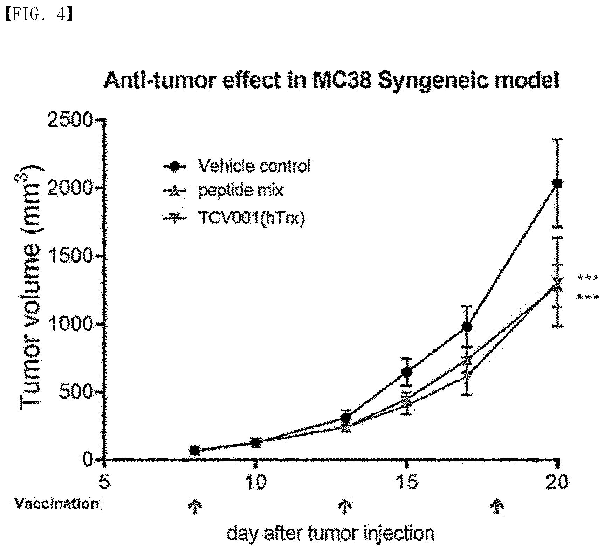

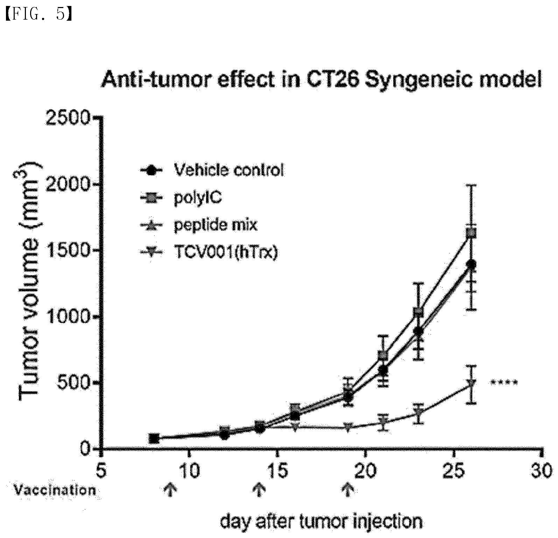

As a result, as shown in FIG. 4 and FIG. 5, in case of the MC38 model, TCV001 and peptide mixture showed the equivalent tumor regression result. This was statistically significantly shown compared to the vehicle control. In case of the CT26 model, strong anticancer efficacy was shown in the TCV001, and the tumor was reduced statistically significantly compared to the peptide mixture.

As the sample of the TCV001 used in the mouse homogeneous anticancer model, the neoantigen and the recombinant protein for antigen delivery having the structure of Table 3 were mixed and used. For the CT26 model, pc0885(CT4), pc0886(CT5), and pc0888(CT24) were mixed and used, and for the MC38 model, pc1304(Cpne1), pc1307(Irgq), and pc1308(Aatf) were mixed, and used as an antigen. In case of the peptide mixture, the synthetic peptide antigen having the amino acid sequence of Table 1 was mixed and used. For the CT26 model, CT4, CT5, and CT24 were mixed and used, and for the MC38 model, Cpne1, Irgq, and Aatf were mixed and used as an antigen.

### 5-2. TIL analysis

In order to measure the activity of the lymphocyte present in the tumor, FACS analysis was performed (4 mice/group). When the tumor of the mouse was grown to an appropriate size, it was removed and then chopping was carried out with scissors. Shaking was performed using collagenase at 37°C 200rpm for 1 hour. After centrifugation and washing with HBSS, using fercoll, the lymphocyte part was obtained. The obtained lymphocyte was treated with PMA, ionomycin, and a transport inhibitor, and then cultured in a 37°C, 5% CO2 incubator for 4 hours. After performing permeabilization and fixation, CD45, CD4, CD8, and IFN-r antibodies were stained for 15 minutes. After passing through a washing process once, FACS analysis was conducted.

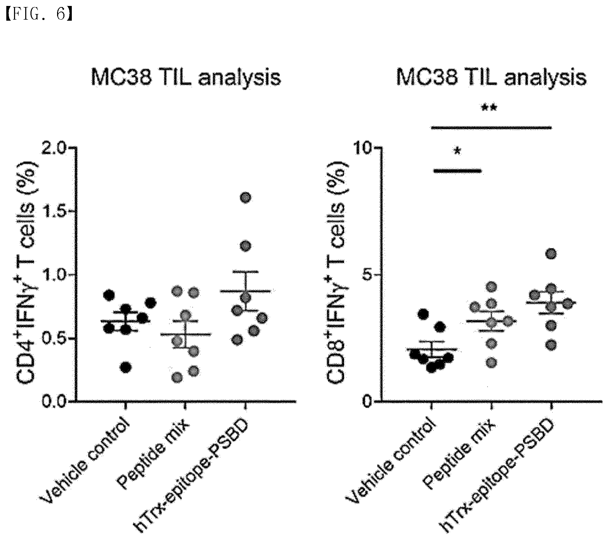

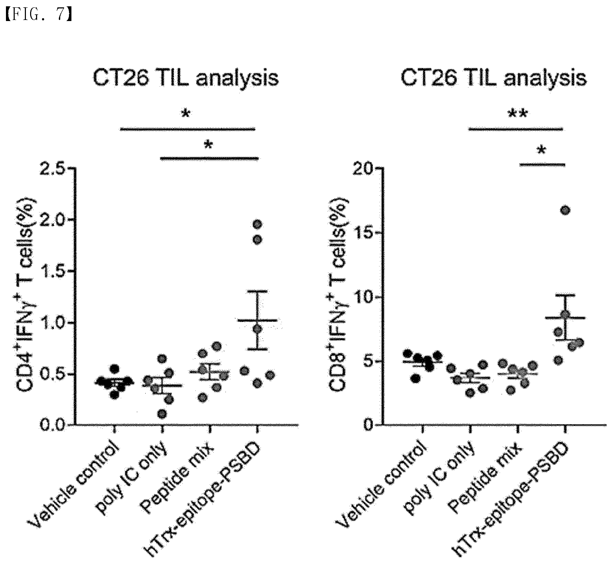

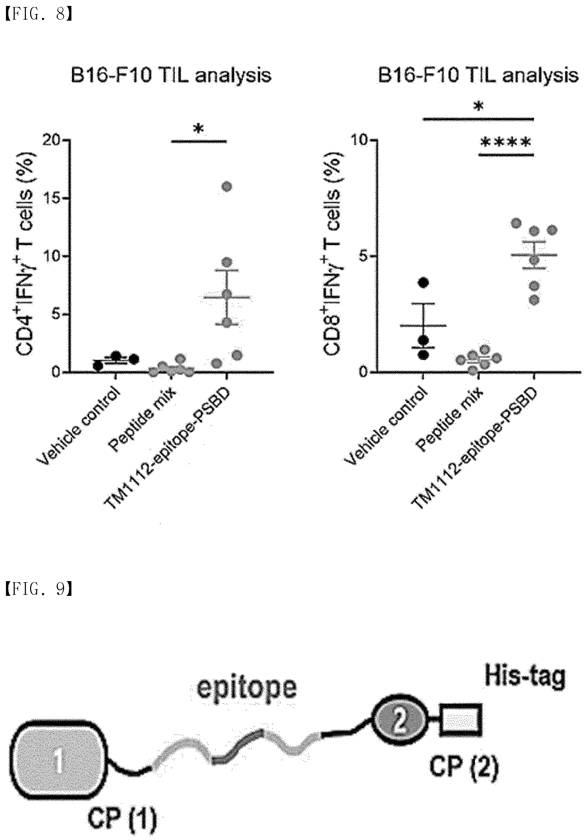

As a result, as shown in FIG. 6, and FIG. 7 and FIG. 8, the hTrx-epitope-PSBD and TM1112-epitope-PSBD increased the activated T cells in TIL compared to the peptide mixture. This result was not shown in all the animal models, but activation of the T cells was increased overall.

As the sample of the hTrx-epitope-PSBD used in the TIL analysis, the neoantigen and the recombinant protein for antigen delivery having the structure of Table 3 were mixed and used. For the CT26 model, pc0885(CT4), pc0886(CT5), and pc0888(CT24) were mixed and used, and for the MC38 model, pc1304(Cpne1), pc1307(Irgq), and pc1308(Aatf) were mixed and used, and for the B16F10 model, pc924(M44), pc923(M30), pc903(M12), and pc933(M21) were mixed and used as an antigen. In case of the peptide mixture, the synthetic peptide antigen having the amino acid sequence of Table 1 was mixed and used. For the CT26 model, CT4, CT5, and CT24 were mixed and used, and for the MC38 model, Cpne1, Irgq, and Aatf were mixed and used as an antigen, and for the B16F10 model, M44, M30, M12, and M21 were mixed and used as an antigen. As the sample of the TM1112-epitope-PSBD, pc0924(M44), pc0923(M30), pc0903(M12), and pc0933(M21) were mixed and used as an antigen.

From the above description, those skilled in the art to which the present invention pertains will be able to understand that the present invention may be implemented in other specific forms without changing the technical spirit or essential characteristics thereof. Therefore, it should be understood that the examples described above are illustrative and not restrictive in all respects. The scope of the present invention should be construed as including all changes or modified forms derived from the meaning and scope of the claims to be described later and equivalent concepts thereof rather than the detailed description.

**Claims**

1. A fusion protein, comprising

   a peptide antigen, and
   a carrier protein linked to the N-terminus, C-terminus, or both of the peptide antigen.

2. The fusion protein according to claim 1,
   comprising a peptide antigen, a fist carrier protein linked to the N-terminus of the peptide antigen, and a second carrier protein linked to the C-terminus of the peptide antigen.

3. The fusion protein according to claim 2,
   wherein in the fusion protein, an affinity tag is linked to the N-terminus, C-terminus, or both thereof.

4. The fusion protein according to claim 2,
   wherein a linker is present between the peptide antigen and the first carrier protein, or a linker is present between the peptide antigen and the second carrier protein, or both.

5. The fusion protein according to claim 1,
   wherein the peptide antigen comprises a T cell epitope.

6. The fusion protein according to claim 1,
   wherein the peptide antigen comprises a T cell epitope, derived from a tumor antigen, an antigen of an infection source, an autoantigen, or an allergy-inducing antigen.

7. The fusion protein according to claim 6,
   wherein the tumor antigen comprises a tumor-associated antigen (TAA), a tumor-specific antigen (TSA) or a tumor-derived neoantigen.

8. The fusion protein according to claim 7,
   wherein the tumor-derived neoantigen comprises a mutation expressed specifically in a cancer cell.

9. The fusion protein according to claim 7,
   wherein the tumor-associated antigen (TAA) is CT (Cancer-testis) antigen, EGFR, M12, M20, M21, M30, M44, Ova, Melan-A, PSMA(Prostate Specific Membrane Antigen), Survivin, MAGE-A, ADAbp (adenosine deaminase-binding protein), cyclophilin b, gp100, CRC (Colorectal associated antigen)-C017-1 A/GA733, CEA (carcinoembryonic antigen), CAP-1, CAP-2, etv6, AML1, PSA (Prostate Specific Antigen), PSA-1, PSA-2, PSA-3, MAGE (melanoma antigen E), GAGE (G antigen), BAGE (B melanoma antigen), RAGE (renal tumor antigen), LAGE (L antigen), NAG, GnT-V, MUM-1, CDK4, p53, tyrosinase, Muc1 (mucin1), HER2/neu, p21ras, N-RAS, K-RAS, RCAS1, a -fetoprotein, E-cadherin, $\alpha$-catenin, $\beta$-catenin, $\gamma$-catenin, p120ctn, PRAME, NY-ESO-1, TRP2, Mammaglobin-A, metallopanstimulin-1 (MPS-1), cytochrome P450 isoform 1B1, 90K/Mac-2 binding protein, Ep-CAM (MK-1), HSP-70, hTERT (TRT), LEA, TAGE-1, 5T4, gp70, SCP-1, c-myc, cyclin B1, MDM2, p62, Koc, IMP1, TA90, OA1, CT-7, HOM-MEL-40/SSX-2, SSX-1, SSX-4, HOM-TES-14/SCP-1, HOM-TES-85, HDAC5, MBD2, TRIP4, NY-CO-45, KNSL6, HIP1R, Seb4D, KIAA1416, IMP1, 90K/Mac-2 binding protein, MDM2, or LMNA.

10. The fusion protein according to claim 6,
    wherein the antigen of the infection source is an antigen derived from a virus, a bacterium, a parasite, or a fungus.

11. The fusion protein according to claim 1,
    wherein the carrier protein is a protein which improves recombinant expression of a peptide antigen, or improves purification of a peptide antigen.

12. The fusion protein according to claim 2,
    wherein the first carrier protein is a protein which improves recombinant expression of a peptide antigen, and the second carrier protein improves improvement of a peptide antigen.

13. The fusion protein according to claim 2,
    wherein the first carrier protein and the second carrier protein are same or different, and are at least one kind of NDPK (nucleoside diphosphate kinase B), CSTA (Cystatin-A), Trx (Thioredoxin), RPL7Am (50S ribosomal protein L7Ae), Samp2a (Small archaeal modifier protein 2), TE (Tenascin), TM1112 (*Thermotoga maritima* Cupin_3 domain-containing protein), TrxA (Thioredoxin 1), TTrx (*Thermosipho africanus* Thioredoxin), PSBD (peripheral subunit-binding domain), or fragments thereof, respectively.

14. The fusion protein according to claim 3,
    wherein the affinity tag is His or streptavidin.

15. The fusion protein according to claim 4,

    wherein the linker is (GS)n, (G$_2$S)n, (G$_3$S)n, (G$_4$S)n, Gn, LE, SSGG or GGGGSGGGGG
    (wherein, G is Gly, and S is Ser, and L is Leu, and E is Glu, and n is an integer of at least 1).

16. The fusion protein according to claim 1,
    wherein the fusion protein has a size of 30kDa or less.

17. A nucleic acid molecule encoding the fusion protein of any one claim of claim 1 to claim 16.

18. An expression vector comprising the nucleic acid molecule of claim 17.

19. A cell transformed with the expression vector of claim 18.

20. An immunogenic composition, comprising

    the fusion protein of any one claim of claim 1 to claim 16;
    a nucleic acid molecule encoding the fusion protein;
    an expression vector comprising the nucleic acid molecule; or
    a cell transformed with the expression vector.

21. The immunogenic composition according to claim 20, further comprising an adjuvant.

【FIG. 1】

【FIG. 2】

## Result of SE-HPLC

| | RT | Area | % Area | Height |
|---|---|---|---|---|
| 1 | 3.689 | 28151 | 0.27 | 1343 |
| 2 | 5.584 | 10535208 | 99.73 | 384202 |

【FIG. 3】

【FIG. 4】

Anti-tumor effect in MC38 Syngeneic model

【FIG. 5】

Anti-tumor effect in CT26 Syngeneic model

【FIG. 6】

【FIG. 7】

CT26 TIL analysis

CT26 TIL analysis

【FIG. 8】

B16-F10 TIL analysis

B16-F10 TIL analysis

【FIG. 9】

## INTERNATIONAL SEARCH REPORT

| International application No. |
| --- |
| **PCT/KR2023/011095** |

**A. CLASSIFICATION OF SUBJECT MATTER**

**C07K 14/00**(2006.01)i; **C07K 14/47**(2006.01)i; **C12N 15/62**(2006.01)i; **A61K 39/00**(2006.01)i; **A61P 37/00**(2006.01)i

According to International Patent Classification (IPC) or to both national classification and IPC

**B. FIELDS SEARCHED**

Minimum documentation searched (classification system followed by classification symbols)

C07K 14/00(2006.01); A61K 38/00(2006.01); A61K 39/00(2006.01); A61P 35/00(2006.01); C07K 1/06(2006.01); C07K 14/435(2006.01); C07K 14/47(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항원(antigen), 면역원성(immunogenicity), 펩티드(peptide), 캐리어 단백질(carrier protein), N-말단(N-terminal), C-말단(C-terminal)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2018-0129899 A (NEON THERAPEUTICS INC.) 05 December 2018 (2018-12-05)<br>See claims 1, 40, 41, 76, 78, 87 and 162; and paragraphs [0161], [0277], [0305], [0327], [0359] and [0381]. | 1-21 |
| A | KR 10-2014-0029446 A (COMPUGEN LTD.) 10 March 2014 (2014-03-10)<br>See entire document. | 1-21 |
| A | WO 2019-197567 A2 (ENTEROME S.A.) 17 October 2019 (2019-10-17)<br>See entire document. | 1-21 |
| A | WO 2021-094562 A2 (ENTEROME S.A.) 20 May 2021 (2021-05-20)<br>See entire document. | 1-21 |
| A | KR 10-2007-0027502 A (WYETH LLC) 09 March 2007 (2007-03-09)<br>See entire document. | 1-21 |

☐ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **30 October 2023** | **31 October 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/011095** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2023/011095**

| Patent document cited in search report | | | Publication date (day/month/year) | Patent family member(s) | | | Publication date (day/month/year) |
|---|---|---|---|---|---|---|---|
| KR | 10-2018-0129899 | A | 05 December 2018 | CN | 109310739 | A | 05 February 2019 |
| | | | | CN | 115558030 | A | 03 January 2023 |
| | | | | EP | 3436048 | A1 | 06 February 2019 |
| | | | | JP | 2019-513373 | A | 30 May 2019 |
| | | | | JP | 2022-116237 | A | 09 August 2022 |
| | | | | KR | 10-2022-0163523 | A | 09 December 2022 |
| | | | | US | 2019-0307868 | A1 | 10 October 2019 |
| | | | | WO | 2017-173321 | A1 | 05 October 2017 |
| KR | 10-2014-0029446 | A | 10 March 2014 | CN | 103596974 | A | 19 February 2014 |
| | | | | CN | 103596974 | B | 31 August 2016 |
| | | | | CN | 105601741 | A | 25 May 2016 |
| | | | | EP | 2697256 | A1 | 19 February 2014 |
| | | | | JP | 2014-517685 | A | 24 July 2014 |
| | | | | JP | 2017-200908 | A | 09 November 2017 |
| | | | | US | 2014-0044641 | A1 | 13 February 2014 |
| | | | | US | 2016-0347816 | A1 | 01 December 2016 |
| | | | | US | 2018-0194829 | A1 | 12 July 2018 |
| | | | | US | 2022-0048972 | A1 | 17 February 2022 |
| | | | | US | 9409987 | B2 | 09 August 2016 |
| | | | | WO | 2012-140627 | A1 | 18 October 2012 |
| WO | 2019-197567 | A2 | 17 October 2019 | CN | 112118863 | A | 22 December 2020 |
| | | | | EP | 3773689 | A2 | 17 February 2021 |
| | | | | EP | 3773689 | B1 | 09 November 2022 |
| | | | | EP | 4169528 | A1 | 26 April 2023 |
| | | | | JP | 2021-520818 | A | 26 August 2021 |
| | | | | US | 2021-0113678 | A1 | 22 April 2021 |
| | | | | WO | 2019-197567 | A3 | 28 November 2019 |
| WO | 2021-094562 | A2 | 20 May 2021 | CN | 114746112 | A | 12 July 2022 |
| | | | | EP | 4021487 | A2 | 06 July 2022 |
| | | | | JP | 2023-501204 | A | 18 January 2023 |
| | | | | US | 2022-0323561 | A1 | 13 October 2022 |
| | | | | WO | 2021-094562 | A3 | 30 September 2021 |
| KR | 10-2007-0027502 | A | 09 March 2007 | CN | 100984676 | A | 20 June 2007 |
| | | | | EP | 1701968 | A2 | 20 September 2006 |
| | | | | EP | 1701968 | B1 | 03 June 2015 |
| | | | | JP | 2008-505052 | A | 21 February 2008 |
| | | | | JP | 4764830 | B2 | 07 September 2011 |
| | | | | KR | 10-1157694 | B1 | 20 June 2012 |
| | | | | US | 2007-0134762 | A1 | 14 June 2007 |
| | | | | WO | 2005-058940 | A2 | 30 June 2005 |
| | | | | WO | 2005-058940 | A3 | 26 October 2006 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10023657 B
- WO 9014837 A
- WO 9313302 A
- WO 9219265 A